# EUROPEAN PATENT APPLICATION

(11) **EP 4 772 145 A2**
(43) Date of publication of application: **08.07.2026**
(21) Application number: 26179558.7
(22) Date of filing: 13.01.2022
(51) Int. Cl.: A61F 2/24

(54) **IMPLANTABLE MEDICAL DEVICES**

(30) Priority: 14.01.2021 US 202163137678 P
(62) Divisional of application: 22702370.2
(71) Applicant: Edwards Lifesciences Corporation, Irvine, CA 92614 (US)
(72) Inventor: Hoang, Lien Huong Thi, IRVINE, CA, 92614 (US); Pawar, Sandip Vasant, IRVINE, CA, 92614 (US); Winters, Taylor Michael, IRVINE, CA, 92614 (US)
(74) Representative: Eisenführ Speiser

(57) **Abstract**

Disclosed herein are implantable prosthetic valves having outer sealing member comprising a bulge in the expanded configuration. The bulge can be configured to provide sealing against native anatomy when the valve is implanted and to assist in minimizing a paravalvular leakage. In addition, disclosed herein are methods of making the prosthetic valves.

## Description

### CROSS-REFERENCE TO THE RELATED APPLICATIONS

This application claims the benefit of U.S. Provisional Application No. 63/137,678, filed January 14, 2021, the content of which is incorporated herein by reference in its entirety.

### FIELD

The present disclosure relates to implantable expandable prosthetic devices and methods and apparatuses for such prosthetic devices.

### BACKGROUND

The heart can suffer from various valvular diseases or malformations that result in significant malfunctioning of the heart and ultimately require replacing the native heart valve with an artificial one. Human heart valves, which include the aortic, pulmonary, mitral, and tricuspid valves, function essentially as one-way valves operating in synchronization with the pumping heart. The valves allow blood to flow downstream but block blood from flowing upstream. Diseased heart valves exhibit impairments such as narrowing of the valve or regurgitation, which inhibits the valves' ability to control blood flow. Such impairments reduce the heart's blood-pumping efficiency and can be a debilitating and life-threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open-heart surgery. Replacement valves are being designed to be delivered through minimally invasive procedures and even percutaneous procedures. Such replacement valves often include a tissue-based valve body that is connected to an expandable frame that is then delivered to the native valve's annulus.

These replacement valves are often intended to at least partially block blood flow. However, a problem occurs when blood flows around the valve on the outside of the prosthesis. For example, in the context of replacement heart valves, paravalvular leakage has proven particularly challenging. An additional challenge relates to the ability of such prostheses to be secured relative to intra-luminal tissue, e.g., tissue within any body lumen or cavity, in an atraumatic manner. Further challenges arise when trying to controllably deliver and secure such prostheses in a location such as at a native mitral valve. These replacement valves are often intended to at least partially block blood flow.

Because of the drawbacks associated with conventional open-heart surgery, percutaneous and minimally-invasive surgical approaches are garnering intense attention. In one technique, a prosthetic valve is configured to be implanted in a much less invasive procedure by way of catheterization. For instance, U.S. Pat. Nos. 5,411,522 and 6,730,118, 7,393,360, 7,510,575, and 7,993,394, which are incorporated herein by reference, describe collapsible transcatheter heart valves (THVs) that can be percutaneously introduced in a compressed state on a catheter and expanded in the desired position by balloon inflation or by utilization of a self-expanding frame or stent. In yet another example, U.S. U.S. Publication Nos. 2014/0277390, 2014/0277422, 2014/0277427, and 2015/0328000, and 2019/0328515, which are incorporated herein by reference in their entirety, describe heart valve prostheses for replacing a native mitral valve, including a self-expanding frame with a plurality of anchoring members that are designed be deployed within a body cavity and prevent axial flow of fluid around an exterior of the prosthesis.

However, problems still can occur. For example, in the context of replacement heart valves, paravalvular leakage has proven particularly challenging.

An additional challenge relates to the ability of such prostheses to be secured relative to intra-luminal tissue, e.g., tissue within any body lumen or cavity, in an atraumatic manner.

### SUMMARY

Some of the aspects of the present disclosure relate to textiles. Some aspects disclosed herein are directed to an implantable prosthetic valve comprising: a) an annular frame having an inner surface and an outer surface, an inflow end and an outflow end; wherein the annular frame is compressible and expandable between a radially compressed configuration and a radially expanded configuration; and b) an outer sealing member having a proximal end and a distal end and is mounted circumferentially around a first portion of the outer surface of the annular frame, wherein the first portion of the outer surface having a proximal end and a distal end, wherein the proximal end of the first portion is at the inflow end of the annular frame, and wherein the first portion has a first length extending between the proximal end and the distal end in the radially compressed configuration of the annular frame and a second length extending between the proximal end and the distal end in the radially expanded configuration of the annular frame; wherein the outer sealing member has a length substantially identical to the first length of the first portion; and wherein the outer sealing member is configured to outwardly bulge when the annular frame is in the radially expanded configuration, thereby forming a seal against surrounding tissue when the prosthetic valve is implanted.

In yet further aspects, the outer surface of the annular frame of the disclosed implantable prosthetic valve has a second portion that is free of the outer sealing member, and wherein the second portion extends between the outflow end of the annular frame and the distal end of the first portion.

In some other aspects, the outer sealing member can comprise a mesh layer and a pile layer, wherein the pile layer comprises a plurality of pile yarns extending outwardly from at least a portion of an outer surface of the mesh layer, and wherein an inner surface of the mesh layer is substantially free of the pile yarns and wherein at least a portion of the inner surface of the mesh layer is in substantial contact with at least a portion of the outer surface of the annular frame.

Also disclosed are aspects where the mesh layer can comprise a plurality of warp and weft yarns and comprises a plurality of wales extending along axially across the length of the outer sealing layer and a plurality of courses extending circumferentially along a width of the outer sealing layer, wherein the width of the outer sealing layer is substantially identical to a circumference of the annular frame in the expanded configuration.

In addition, or the alternative, disclosed are aspects where the mesh layer comprises a knit or woven fabric. In some exemplary aspects, the knit fabric is crochet knit and/or warp-knit fabric. Also disclosed are aspects where the pile yarns are arranged to form a looped pile. In still further exemplary and unlimiting aspects, the pile yarns can be cut to form a cut pile.

In addition to, or in the alternative of some aspects disclosed herein disclosed are methods of making the disclosed implantable prosthetic valves. In some aspects, disclosed herein is a method of forming an implantable prosthetic valve comprising: a) providing an annular frame having an inner surface and an outer surface wherein the frame has an inflow end and an outflow end; wherein the annular frame is compressible and expandable between a radially compressed configuration and a radially expanded configuration; b) circumferentially mounting an outer sealing member having a proximal end and a distal end around a first portion of the outer surface of the annular frame, wherein the first portion of the outer surface has a proximal end, and a distal end, wherein the proximal end of the first portion is at the inflow end of the annular frame, and wherein the first portion has a first length extending between the proximal end and the distal end in the radially compressed configuration of the annular frame and a second length extending between the proximal end and the distal end in the radially expanded configuration of the annular frame; wherein the outer sealing member has a length substantially identical to the first length of the first portion; and wherein the outer sealing member is configured to outwardly bulge when the annular frame is in the radially expanded configuration, thereby forming a seal against surrounding tissue when the prosthetic valve is implanted.

Also disclosed are methods where the outer sealing member is knitted to the desired width.

In certain exemplary aspects, the desired width is substantially similar to a circumference of the annular frame in the expanded configuration.

In certain aspects disclosed are methods where the outer sealing member is laser cut at the proximal end and/or distal end.

Also disclosed are methods further comprising an inner skirt mounted on the inner surface of the annular frame, where the inner skirt has an inflow end portion that is secured to the proximal end of the outer sealing member. In such exemplary and unlimiting aspects, the inflow end portion of the inner skirt is wrapped around the inflow end of the frame and overlaps the proximal end portion of the outer sealing member on the outside of the frame.

Additional aspects of the disclosure will be set forth, in part, in the detailed description, figures, and claims which follow, and in part will be derived from the detailed description or can be learned by practice of the invention. It is to be understood that both the foregoing general description and the following detailed description are exemplary and explanatory only and are not restrictive of the invention as disclosed.

### BRIEF DESCRIPTION OF THE DRAWINGS

**FIGURE 1** is a perspective view of a prosthetic heart valve, according to one aspect.
**FIGURE 2** is an enlarged, perspective view of the inflow end portion of the prosthetic heart valve of **FIG.** 1.
**FIGURE 3** is a cross-sectional view of the prosthetic heart valve of **FIG.** 1, showing the attachment of the outer skirt to the inner skirt and the frame.
**FIGURES 4-10** show an exemplary frame of the prosthetic heart valve of **FIG. 1****.**
**FIGURES 11-12** show an exemplary inner skirt of the prosthetic heart valve of **FIG. 1****.**
**FIGURES 13-15** show the assembly of the inner skirt of **FIG. 11** with the frame of **FIG. 4****.**
**FIGURES 16-17** show the assembly of an exemplary leaflet structure.
**FIGURE 18** shows the assembly of commissure portions of the leaflet structure with window frame portions of the frame.
**FIGURES 19-20** show the assembly of the leaflet structure with the inner skirt along a lower edge of the leaflets.
**FIGURES 21A-21B** show a schematic of the compressed and expanded configuration of an exemplary annular frame.
**FIGURE 22** shows a schematic representation of the outer skirt as it can be placed on a compressed and expanded configuration of an exemplary annular frame.
**FIGURE 23** shows a schematic representation of the implantable device in native anatomy in one aspect.
**FIGURE 24** shows an exemplary fabric used in the outer skirt in one aspect.
**FIGURE 25** shows a schematic of an exemplary outer skirt as it to be mounted on an exemplary frame in one aspect.
**FIGURES 26-28** are different views of an exemplary outer skirt of the prosthetic heart valve of **FIG.** 1.
**FIGURES 29-30** how an alternative way of securing an outer skirt to an inner skirt and/or the frame of a prosthetic heart valve.

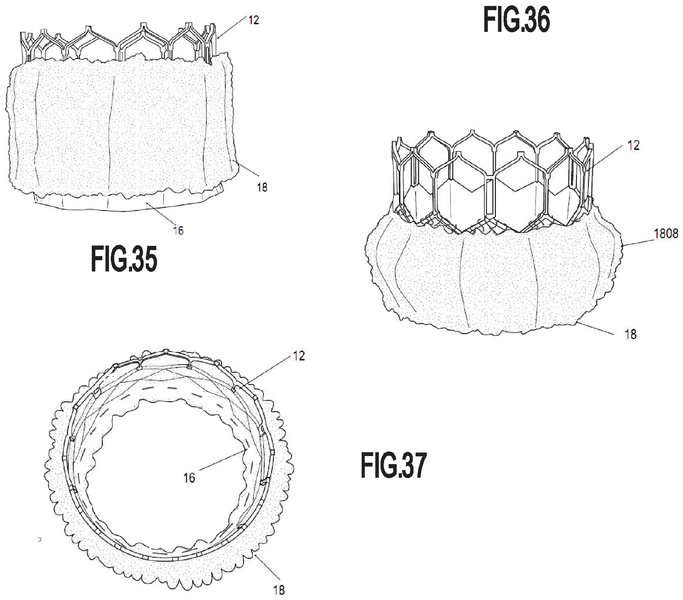

**FIGURES 31-35** show another way of securing an outer skirt to an inner skirt and/or the frame of a prosthetic heart valve.
**FIGURES 36-37** show different views of an exemplary outer skirt of the prosthetic heart valve of **FIG.** 1.
**FIGURES 38-41** show an exemplary fabric used in the outer skirt in one aspect.

### DETAILED DESCRIPTION

The present invention can be understood more readily by reference to the following detailed description, examples, drawings, and claims, and their previous and following description. However, before the present articles, systems, and/or methods are disclosed and described, it is to be understood that this invention is not limited to the specific or exemplary aspects of articles, systems, and/or methods disclosed unless otherwise specified, as such can, of course, vary. It is also to be understood that the terminology used herein is for the purpose of describing particular aspects only and is not intended to be limiting.

The following description of the invention is provided as an enabling teaching of the invention in its best, currently known aspect. To this end, those skilled in the relevant art will recognize and appreciate that many changes can be made to the various aspects of the invention described herein while still obtaining the beneficial results of the present invention. It will also be apparent that some of the desired benefits of the present invention can be obtained by selecting some of the features of the present invention without utilizing other features. Accordingly, those of ordinary skill in the pertinent art will recognize that many modifications and adaptations to the present invention are possible and may even be desirable in certain circumstances and are a part of the present invention. Thus, the following description is again provided as illustrative of the principles of the present invention and not in limitation thereof.

### DEFINITIONS

As used in this application and in the claims, the singular forms "a," "an," and "the" include the plural forms unless the context clearly dictates otherwise. Thus, for example, reference to a "yarn" includes aspects having two or more such yarns unless the context clearly indicates otherwise.

It is also to be understood that the terminology used herein is for the purpose of describing particular aspects only and is not intended to be limiting. As used in the specification and in the claims, the term "comprising" can include the aspects "consisting of" and "consisting essentially of." Additionally, the term "includes" means "comprises."

For the terms "for example," "exemplary," and "such as," and grammatical equivalences thereof, the phrase "and without limitation" is understood to follow unless explicitly stated otherwise.

Ranges can be expressed herein as from "about" one particular value and/or to "about" another particular value. When such a range is expressed, another aspect includes from the one particular value and/or to the other particular value. Similarly, when values are expressed as approximations, by use of the antecedent "about," it will be understood that the particular value forms another aspect. It should be further understood that the endpoints of each of the ranges are significant both in relation to the other endpoint, and independently of the other endpoint.

As used herein, the terms "optional" or "optionally" mean that the subsequently described event or circumstance may or may not occur and that the description includes instances where said event or circumstance occurs and instances where it does not.

Further, the terms "coupled" and "associated" generally mean electrically, electromagnetically, and/or physically (e.g., mechanically or chemically) coupled or linked and does not exclude the presence of intermediate elements between the coupled or associated items.

As used herein, the term or phrase "effective," "effective amount," or "conditions effective to" refers to such amount or condition that is capable of performing the function or property for which an effective amount or condition is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, it should be understood that an appropriate effective amount will be readily determined by one of ordinary skill in the art using only routine experimentation.

The term "fiber" as used herein includes fibers of extreme or indefinite length (i.e., filaments) and fibers of short length (i.e., staple fibers).

As used herein, the term "polyester" refers to a category of polymers that contain the ester functional group in their main chain. Polyesters disclosed herein include naturally occurring chemicals, such as in the cutin of plant cuticles, as well as synthetics produced through step-growth polymerization. In certain examples, the polyesters comprise polyethylene terephthalate (PET) homopolymer and copolymers, polypropylene terephthalate (PPT) homopolymer and copolymers and polybutylene terephthalate (PBT) homopolymer and copolymers, and the like, including those that contain comonomers such as cyclohexane dimethanol, cyclohexane dicarboxylic acid, isophthalic acid, and the like.

The term "polyamide," as utilized herein, is defined to be any long-chain polymer in which the linking functional groups are amide (-CO-NH-) linkages. The term polyamide is further defined to include copolymers, terpolymers, and the like, as well as homopolymers, including blends of two or more polyamides. In some aspects, the plurality of polyamide fibers comprise one or more of nylon 6, nylon 66, nylon 10, nylon 612, nylon 12, nylon 11, or any combination thereof. In other aspects, the plurality of polyamide fibers comprise nylon 6 or nylon 66. In yet other aspects, the plurality of polyamide fibers are nylon 6. In a yet further aspect, the plurality of polyamide fibers are nylon 66.

As defined herein, the term "polyolefin" refers to any class of polymers produced from a simple olefin (also called an alkene with the general formula CₙH₂ₙ) as a monomer. In some aspects, the polyolefins include but are not limited to polyethylene, polypropylene, both homopolymer and copolymers, poly(l-butene), poly(3-methyl-l-butene), poly(4- methyl- 1-pentene) and the like, as well as combinations or mixtures of two or more of the foregoing.

As defined herein, the term "polyurethane" refers to any class of polymers composed of a chain of organic units joined by carbamate (urethane, R₁-O-CO-NR₂-R₃, wherein R1, R2, and R3 are the same or different) links.

As defined herein, the term "polyether" refers to any class of polymers composed of a chain of organic units joined by an ether group.

Throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

A weight percent (wt. %) of a component, unless specifically stated to the contrary, is based on the total weight of the formulation or composition in which the component is included.

It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present. Other words used to describe the relationship between elements or layers should be interpreted in a like fashion (e.g., "between" versus "directly between," "adjacent" versus "directly adjacent," "on" versus "directly on"). As used herein, the term "and/or" includes any and all combinations of one or more of the associated listed items.

It will be understood that, although the terms "first," "second," etc., may be used herein to describe various elements, components, regions, layers, and/or sections. These elements, components, regions, layers, and/or sections should not be limited by these terms. These terms are only used to distinguish one element, component, region, layer, or section from another element, component, region, layer, or a section. Thus, a first element, component, region, layer, or section discussed below could be termed a second element, component, region, layer, or section without departing from the teachings of exemplary aspects.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" can encompass both an orientation of above and below. The device may be otherwise oriented (rotated 90 degrees or at other orientations), and the spatially relative descriptors used herein interpreted accordingly.

As used herein, the term "substantially" means that the subsequently described event or circumstance completely occurs or that the subsequently described event or circumstance generally, typically, or approximately occurs.

Still further, the term "substantially" can in some aspects refer to at least about 80 %, at least about 85 %, at least about 90 %, at least about 91 %, at least about 92 %, at least about 93 %, at least about 94 %, at least about 95 %, at least about 96 %, at least about 97 %, at least about 98 %, at least about 99 %, or about 100 % of the stated property, component, composition, or other condition for which substantially is used to characterize or otherwise quantify an amount.

In other aspects, as used herein, the term "substantially free," when used in the context of a composition or component of a composition that is substantially absent, is intended to indicate that the recited component is not intentionally batched and added to the composition, but can be present as an impurity along with other components being added to the composition. In such aspects, the term "substantially free," is intended to refer to trace amounts that can be present in the batched components, for example, it can be present in an amount that is less than about 1 % by weight, e.g., less than about 0.5 % by weight, less than about 0.1 % by weight, less than about 0.05 % by weight, or less than about 0.01 % by weight of the stated material, based on the total weight of the composition.

As used herein, the term "substantially," in, for example, the context "substantially identical" or "substantially similar" refers to a method or a system, or a component that is at least about 90%, at least about 91%, at least about 92%, at least about 93%, at least about 94%, at least about 95%, at least about 96%, at least about 97%, at least about 98%, at least about 99%, or about 100% by similar to the method, system, or the component it is compared to.

As used herein, the term or phrase "effective," "effective amount," or "conditions effective to" refers to such amount or condition that is capable of performing the function or property for which an effective amount or condition is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, it should be understood that an appropriate effective amount will be readily determined by one of ordinary skill in the art using only routine experimentation. Although the operations of exemplary aspects of the disclosed method may be described in a particular sequential order for convenient presentation, it should be understood that disclosed aspects can encompass an order of operations other than the particular sequential order disclosed. For example, operations described sequentially may, in some cases, be rearranged or performed concurrently. Further, descriptions and disclosures provided in association with one particular aspect are not limited to that aspect and may be applied to any aspect disclosed.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

### IMPLANTABLE MEDICAL DEVICE

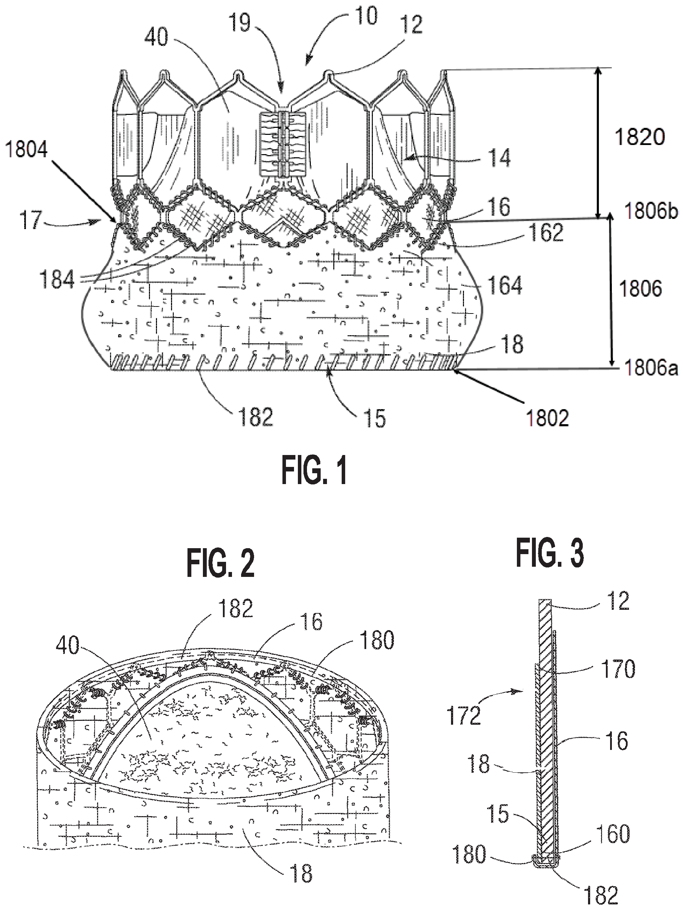

**FIG.** 1 shows a prosthetic heart valve 10, according to one aspect. The illustrated prosthetic valve is adapted to be implanted in the native aortic annulus, although in other aspects, it can be adapted to be implanted in the other native annuluses of the heart (e.g., the pulmonary, mitral, and tricuspid valves). The prosthetic valve can also be adapted to be implanted in other tubular organs or passageways in the body. The prosthetic valve **10** can have four main components: a stent or frame **12,** a valvular structure **14,** an inner skirt **16,** and an exemplary perivalvular outer sealing member or outer skirt **18.** The exemplary prosthetic valve **10** has an inflow end portion **15,** an intermediate portion **17,** and an outflow end portion **19.** The outer sealing member **18** has a proximal end **1802** and a distal end **1804** and is mounted circumferentially around a first portion of the outer surface of the annular frame **12,** wherein the first portion **1806** of the outer surface has a proximal end and a distal end, wherein the proximal end of the first portion is at the inflow end **15** of the annular frame **12.** In aspects disclosed herein, for example, the first portion **1806** of the annular frame can be between the inflow end **15** of the annular frame and the beginning of the intermediate portion **17.** The first portion, **1806,** is also defined by a proximal end **1806a** and a distal end **1806b.**

In still further aspects, the annular frame also has a second portion **1820** that is free of the outer sealing member and extends between the outflow end **19** of the annular frame and the distal end of the first portion **1806b.**

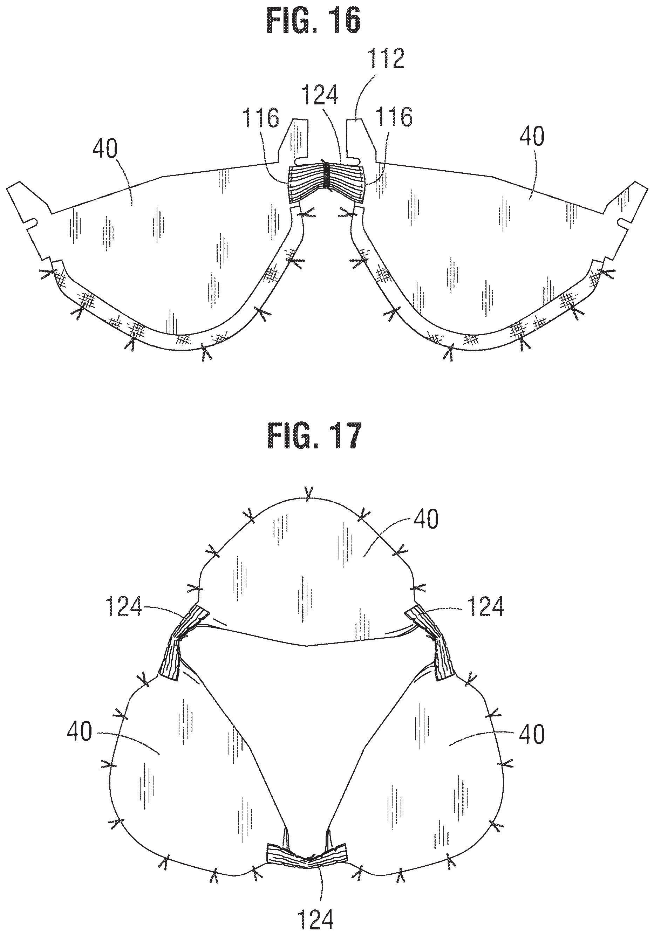

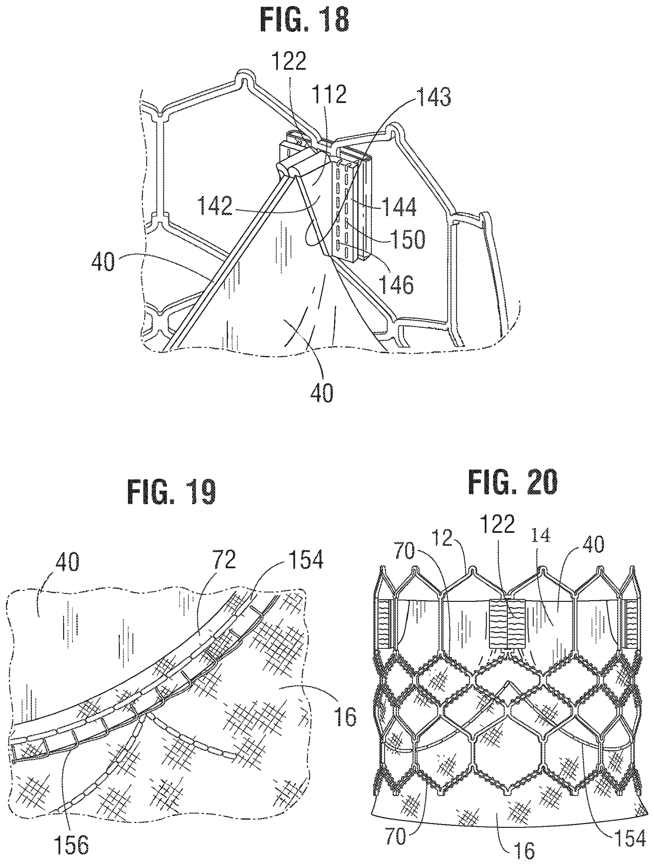

The valvular structure **14** can comprise three leaflets **40 (****FIG. 17****),** collectively forming a leaflet structure, which can be arranged to collapse in a tricuspid arrangement. The lower edge of leaflet structure **14** desirably has an undulating, curved scalloped shape (suture line **154** shown in **FIG. 20** tracks the scalloped shape of the leaflet structure). By forming the leaflets with this scalloped geometry, stresses on the leaflets are reduced, which in turn improves the durability of the prosthetic valve. Moreover, by virtue of the scalloped shape, folds and ripples at the belly of each leaflet (the central region of each leaflet), which can cause early calcification in those areas, can be eliminated or at least minimized. The scalloped geometry also reduces the amount of tissue material used to form leaflet structure, thereby allowing a smaller, more even crimped profile at the inflow end of the prosthetic valve. The leaflets **40** can be formed of pericardial tissue (e.g., bovine pericardial tissue), biocompatible synthetic materials, or various other suitable natural or synthetic materials as known in the art and described in U.S. Pat. No. 6,730,118, which is incorporated by reference herein.

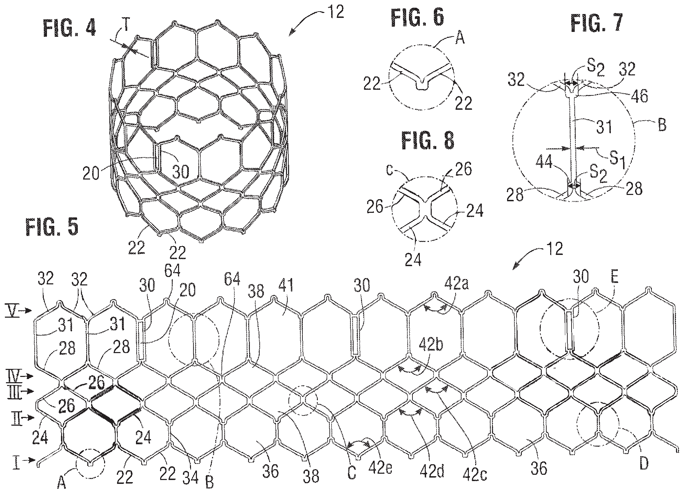

The bare frame **12** is shown in **FIG. 4****.** Frame **12** can be formed with a plurality of circumferentially spaced slots or commissure windows, **20** (three are shown in the illustrated and unlimiting aspect) that are adapted to mount the commissures of the valvular structure **14** to the frame, as described in greater detail below. The frame **12** can be made of any of various suitable plastically-expandable materials (e.g., stainless steel, etc.) or self-expanding materials (e.g., nickel titanium alloy (NiTi), such as nitinol) as known in the art. When constructed of a plastically-expandable material, frame **12** (and thus the prosthetic valve **10)** can be crimped to a radially collapsed configuration on a delivery catheter and then expanded at the implantation site by an inflatable balloon or equivalent expansion mechanism. When constructed of a self-expandable material, frame **12** (and thus the prosthetic valve **10)** can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame **12** include, without limitation, stainless steel, biocompatible, high-strength alloys (e.g., a cobalt-chromium or a nickel-cobalt-chromium alloys), polymers, or combinations thereof. In particular aspects, frame **12** is made of a nickel-cobalt-chromium-molybdenum alloy, such as MP35N^{®} alloy (SPS Technologies, Jenkintown, Pa.), which is equivalent to UNS R30035 alloy (covered by ASTM F562-02). MP35N^{®} alloy/UNS R30035 alloy comprises 35% nickel, 35% cobalt, 20% chromium, and 10% molybdenum by weight. It has been found that the use of MP35N^{®} alloy to form frame **12** provides superior structural results over stainless steel. In particular, when MP35N^{®} alloy is used as the frame material, less material is needed to achieve the same or better performance in radial and crush force resistance, fatigue resistance, and corrosion resistance. Moreover, since less material is required, the crimped profile of the frame can be reduced, thereby providing a lower profile prosthetic valve assembly for percutaneous delivery to the treatment location in the body.

Referring to **FIGS. 4** and **5****,** frame **12** in the illustrated aspect comprises a first, lower row I of angled struts **22** arranged end-to-end and extending circumferentially at the inflow end of the frame; a second row II of circumferentially extending angled struts **24;** a third row III of circumferentially extending, angled struts **26;** a fourth row IV of circumferentially extending angled struts **28;** and a fifth row V of circumferentially extending, angled struts **32** at the outflow end of the frame. A plurality of substantially straight axially extending struts **34** can be used to interconnect the struts **22** of the first row I with the struts **24** of the second row II. The fifth row V of angled struts **32** are connected to the fourth row IV of angled struts **28** by a plurality of axially extending window frame portions **30** (which define the commissure windows **20)** and a plurality of axially extending struts **31.** Each axial strut **31** and each frame portion **30** extends from a location defined by the convergence of the lower ends of two angled struts **32** to another location defined by the convergence of the upper ends of two angled struts **28.** **FIGS. 6, 7, 8****,** **9,** and **10** are enlarged views of the portions of frame **12** identified by letters A, B, C, D, and E, respectively, in **FIG. 5****.**

Each commissure window frame portion **30** mounts a respective commissure of the leaflet structure **14.** As can be seen, each frame portion **30** is secured at its upper and lower ends to the adjacent rows of struts to provide a robust configuration that enhances fatigue resistance under cyclic loading of the prosthetic valve compared to known, cantilevered struts for supporting the commissures of the leaflet structure. This configuration enables a reduction in the frame wall thickness to achieve a smaller crimped diameter of the prosthetic valve. In certain aspects, the thickness T of frame **12 (****FIG. 4****)** measured between the inner diameter and outer diameter is about 0.48 mm or less.

The struts and frame portions of the frame collectively define a plurality of open cells of the frame. At the inflow end of frame **12,** struts **22,** struts **24,** and struts **34** define a lower row of cells defining openings **36.** The second, third, and fourth rows of struts **24, 26,** and **28** define two intermediate rows of cells defining openings **38.** The fourth and fifth rows of struts **28** and **32,** along with frame portions **30** and struts **31,** define an upper row of cells defining openings **40.** The openings **41** are relatively large and are sized to allow portions of the leaflet structure **14** to protrude, or bulge, into and/or through the openings **40** when the frame **12** is crimped in order to minimize the crimping profile.

As best shown in **FIG. 7****,** the lower end of the strut **31** is connected to two struts **28** at a node or junction **44,** and the upper end of the strut **31** is connected to two struts **32** at a node or junction **46.** The strut **31** can have a thickness S₁ that is less than the thicknesses S₂ of the junctions **44, 46.** The junctions **44, 46,** along with junctions **64,** prevent full closure of openings **40.** The geometry of the struts **31,** and junctions **44, 46,** and **64** assists in creating enough space in openings **41** in the collapsed configuration to allow portions of the prosthetic leaflets to protrude or bulge outwardly through openings. This allows the prosthetic valve to be crimped to a relatively smaller diameter than if all of the leaflet material were constrained within the crimped frame.

Frame **12** is configured to reduce, prevent, or minimize possible over-expansion of the prosthetic valve at a predetermined balloon pressure, especially at the outflow end portion of the frame, which supports the leaflet structure **14.** In one aspect, the frame is configured to have relatively larger angles **42a, 42b, 42c, 42d, 42e** between struts, as shown in **FIG. 5****.** The larger the angle, the greater the force required to open (expand) the frame. As such, the angles between the struts of the frame can be selected to limit the radial expansion of the frame at a given opening pressure (e.g., inflation pressure of the balloon). In some exemplary aspects, these angles are at least 110 degrees or greater when the frame is expanded to its functional size, and even more particularly, these angles are up to about 120 degrees when the frame is expanded to its functional size.

In addition, the inflow **15** and outflow **19** ends of a frame generally tend to over-expand more so than the middle portion of the frame due to the "dog-boning" effect of the balloon used to expand the prosthetic valve. To protect against over-expansion of the leaflet structure **14,** the leaflet structure desirably is secured to the frame **12** below the upper row of struts **32,** as best shown in **FIG. 1****.** Thus, in the event that the outflow end of the frame is over-expanded, the leaflet structure is positioned at a level below where over-expansion is likely to occur, thereby protecting the leaflet structure from over-expansion.

In some aspects, in a prosthetic valve construction, portions of the leaflets can protrude longitudinally beyond the outflow end of the frame when the prosthetic valve is crimped if the leaflets are mounted too close to the distal end of the frame. If the delivery catheter on which the crimped prosthetic valve is mounted includes a pushing mechanism or stop member that pushes against or abuts the outflow end of the prosthetic valve (for example, to maintain the position of the crimped prosthetic valve on the delivery catheter), the pushing member or stop member can damage the portions of the exposed leaflets that extend beyond the outflow end of the frame. Another benefit of mounting the leaflets at a location spaced away from the outflow end of the frame is that when the prosthetic valve is crimped on a delivery catheter, the outflow end of the frame **12** rather than the leaflets **40** is the proximal-most component of the prosthetic valve **10.** As such, if the delivery catheter includes a pushing mechanism or stop member that pushes against or abuts the outflow end of the prosthetic valve, the pushing mechanism or stop member contacts the outflow end of the frame, and not leaflets **40,** so as to avoid damage to the leaflets.

Also, as can be seen in **FIG. 5****,** the openings **36** of the lowermost row of openings in the frame are relatively larger than the openings **38** of the two intermediate rows of openings. This allows the frame, when crimped, to assume an overall tapered shape that tapers from a maximum diameter at the outflow end of the prosthetic valve to a minimum diameter at the inflow end of the prosthetic valve. When crimped, frame **12** has a reduced diameter region extending along a portion of the frame adjacent the inflow end of the frame that generally corresponds to the region of the frame covered by the outer skirt **18.** In some aspects, the reduced diameter region is reduced compared to the diameter of the upper portion of the frame (which is not covered by the outer skirt) such that the outer skirt **18** does not increase the overall crimp profile of the prosthetic valve. When the prosthetic valve is deployed, the frame can expand to the generally cylindrical shape shown in **FIG. 4****.** In one example, the frame of a 26-mm prosthetic valve, when crimped, had a first diameter of 14 French at the outflow end of the prosthetic valve and a second diameter of 12 French at the inflow end of the prosthetic valve.

The main functions of the inner skirt **16 are** to assist in securing the valvular structure **14** to the frame **12** and to assist in forming a good seal between the prosthetic valve and the native annulus by blocking the flow of blood through the open cells of the frame **12** below the lower edge of the leaflets. The inner skirt **16** desirably comprises a tough, tear-resistant material such as polyethylene terephthalate (PET), although various other synthetic materials or natural materials (e.g., pericardial tissue) can be used. The thickness of the skirt desirably is less than about 0.15 mm (about 6 mil), and desirably less than about 0.1 mm (about 4 mil), and even more desirably about 0.05 mm (about 2 mil). In certain exemplary and unlimiting aspects, inner skirt **16** can have a variable thickness, for example, the skirt can be thicker at at least one of its edges than at its center. In one implementation, inner **skirt 16** can comprise a PET skirt having a thickness of about 0.07 mm at its edges and about 0.06 mm at its center. The thinner skirt can provide for better crimping performances while still providing good perivalvular sealing.

The inner skirt **16** can be secured to the inside of frame **12** via sutures **70,** as shown in **FIG. 20****.** Valvular structure **14** can be attached to the skirt via one or more reinforcing strips **72 (****FIG. 19****)** (which collectively can form a sleeve), for example, thin, PET reinforcing strips, discussed below, which enables a secure suturing and protects the pericardial tissue of the leaflet structure from tears. Valvular structure **14** can be sandwiched between skirt **16** and the thin PET strips **72,** as shown in **FIG. 19****.** Sutures **154,** which secure the PET strip and the leaflet structure **14** to skirt **16,** can be any suitable suture, such as Ethibond Excel^{®} PET suture (Johnson & Johnson, New Brunswick, N.J.). Sutures **154** desirably track the curvature of the bottom edge of leaflet structure **14,** as described in more detail below.

In certain aspects, the fabric of the inner skirts can comprise a weave of warp and weft fibers that extend perpendicularly to each other and with one set of fibers extending longitudinally between the upper and lower edges of the skirt. When the metal frame to which the fabric of the inner skirt is secured is radially compressed, the overall axial length of the frame increases. Unfortunately, an inner skirt with limited elasticity cannot elongate along with the frame and therefore tends to deform the struts of the frame and to prevent uniform crimping.

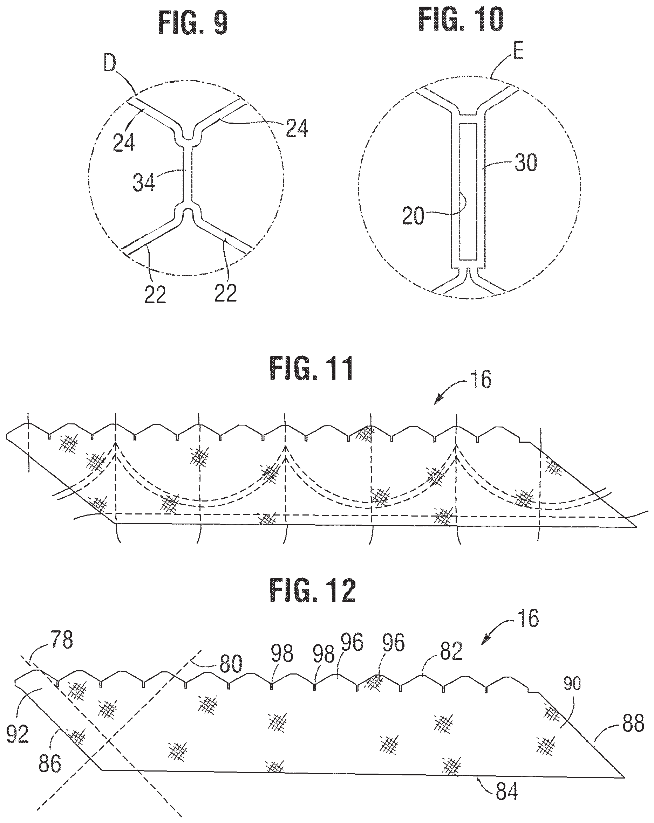

Referring to **FIG. 12****,** in contrast to known fabric skirts, the exemplary inner skirt **16** as disclosed herein can be woven from a first set of fibers, or yarns or strands, **78** and a second set of fibers, or yarns or strands, **80,** both of which are non-perpendicular to the upper edge **82** and the lower edge **84** of the skirt. In particular aspects, the first set of fibers **78** and the second set of fibers **80** extend at angles of about 45 degrees relative to the upper and lower edges **82, 84.** Alternatively, the first set of fibers **78** and the second set of fibers **80** extend at angles other than about 45 degrees relative to the upper and lower edges **82, 84,** e.g., at angles of 15 and 75 degrees, respectively, or 30 and 60 degrees, respectively, relative to the upper and lower edges **82, 84.** For example, the inner skirt **16** can be formed by weaving the fibers at 45 degree angles relative to the upper and lower edges of the fabric. Alternatively, the inner skirt **16** can be diagonally cut (cut on a bias) from a vertically woven fabric (where the fibers extend perpendicularly to the edges of the material) such that the fibers extend at 45-degree angles relative to the cut upper and lower edges of the skirt. As further shown in **FIG. 12****,** the opposing short edges **86, 88** of the inner skirt can be, for example, non-perpendicular to the upper and lower edges **82,** 84. In another example, the short edges **86, 88** can extend at angles of about 45 degrees relative to the upper and lower edges and therefore are aligned with the first set of fibers 78. Therefore the overall general shape of the inner skirt is that of a rhomboid or parallelogram. Without wishing to be bound by any theory, it is noted that the 45 degree angle orientation can provide dimensional compliance to a rigid woven cloth as the inner skirt sees dimensional changes during crimping and expansion.

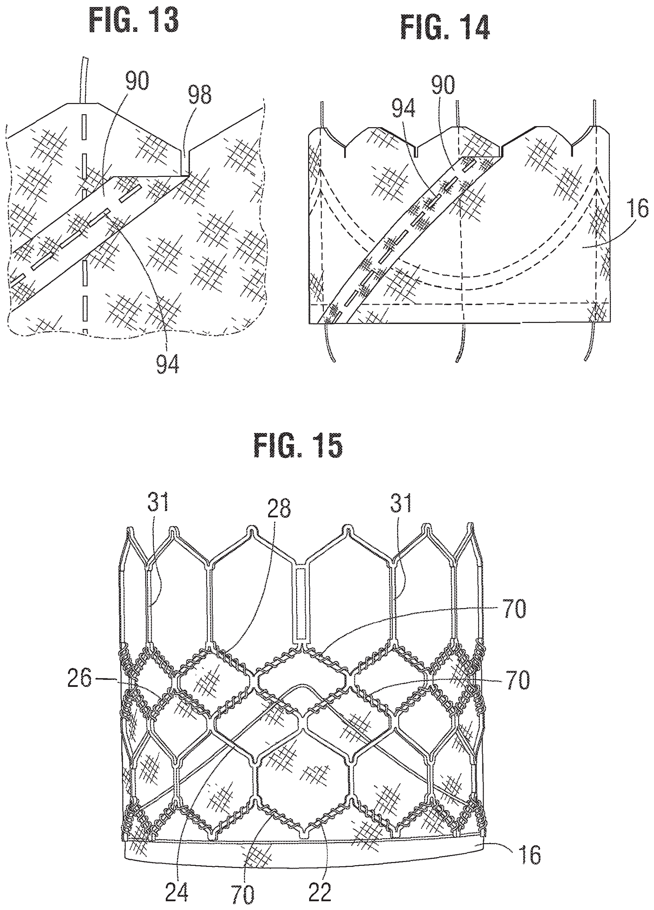

**FIGS. 13** and **14** show the inner skirt **16** after opposing short edge portions **90, 92** have been sewn together to form the annular shape of the skirt. As shown, the edge portion **90** can be placed in an overlapping relationship relative to the opposite edge portion **92,** and the two edge portions can be sewn together with a diagonally extending suture line **94** that is parallel to short edges **86, 88.** The upper edge portion of the inner skirt **16** can be formed with a plurality of projections **96** that define an undulating shape that generally follows the shape or contour of the fourth row of struts **28** immediately adjacent the lower ends of axial struts 31. In this manner, as best shown in **FIG. 15****,** the upper edge of the inner skirt **16** can be tightly secured to struts **28** with sutures 70. The inner skirt **16** can also be formed with slits **98** to facilitate attachment of the skirt to the frame. Slits **98** are dimensioned so as to allow an upper edge portion of the inner skirt **16** to be partially wrapped around struts **28** and to reduce stresses in the skirt during the attachment procedure. For example, in the illustrated aspect, the inner skirt **16** is placed on the inside of frame **12,** and an upper edge portion of the skirt is wrapped around the upper surfaces of struts **28** and secured in place with sutures **70.** Wrapping the upper edge portion of the inner skirt **16** around struts **28** in this manner provides for a stronger and more durable attachment of the skirt to the frame. The inner skirt **16** can also be secured to the first, second, and/or third rows of struts **22, 24,** and **26,** respectively, with sutures **70.**

Due to the angled orientation of the fibers relative to the upper and lower edges, the inner skirt can undergo greater elongation in the axial direction (i.e., in a direction from the upper edge **82** to the lower edge **84).** Thus, when the metal frame **12** is crimped, the inner skirt **16** can elongate in the axial direction along with the frame and therefore provide a more uniform and predictable crimping profile. Each cell of the metal frame in the illustrated aspect includes at least four angled struts that rotate towards the axial direction on crimping (e.g., the angled struts become more aligned with the length of the frame). The angled struts of each cell function as a mechanism for rotating the fibers of the skirt in the same direction of the struts, allowing the skirt to elongate along the length of the struts. This allows for greater elongation of the skirt and avoids undesirable deformation of the struts when the prosthetic valve is crimped.

In addition, the spacing between the woven fibers or yarns can be increased to facilitate elongation of the skirt in the axial direction. For example, for a PET inner skirt **16** formed from 20-denier yarn, the yarn density can be about 15% to about 30% lower than in a typical PET skirt. In some examples, the yarn spacing of the inner skirt **16** can be from about 60 yarns per cm (about 155 yarns per inch) to about 70 yarns per cm (about 180 yarns per inch), such as about 63 yarns per cm (about 160 yarns per inch), whereas in a typical PET skirt the yarn spacing can be from about 85 yarns per cm (about 217 yarns per inch) to about 97 yarns per cm (about 247 yarns per inch). The oblique edges **86, 88** promote a uniform and even distribution of the fabric material along an inner circumference of the frame during crimping so as to reduce or minimize bunching of the fabric to facilitate uniform crimping to the smallest possible diameter. Additionally, cutting diagonal sutures in a vertical manner may leave loose fringes along the cut edges. The oblique edges **86, 88** help minimize this from occurring. Compared to the construction of a typical skirt (fibers running perpendicularly to the upper and lower edges of the skirt), the construction of the inner skirt **16** avoids undesirable deformation of the frame struts and provides more uniform crimping of the frame.

In alternative aspects, the inner skirt can be formed from woven elastic fibers that can stretch in the axial direction during crimping of the prosthetic valve. The warp and weft fibers can run perpendicularly and parallel to the upper and lower edges of the skirt, or alternatively, they can extend at angles between 0 and 90 degrees relative to the upper and lower edges of the skirt, as described above.

The inner skirt **16** can be sutured to frame **12** at locations away from the suture line **154** so that the skirt can be more pliable in that area. This configuration can avoid stress concentrations at the suture line **154,** which attaches the lower edges of the leaflets to the inner skirt **16.**

As noted above, the leaflet structure **14** in the illustrated aspect includes three flexible leaflets **40** (although a greater or a smaller number of leaflets can be used). Additional information regarding the leaflets, as well as additional information regarding inner skirt material, can be found, for example, in U.S. Patent Application Publication No. 2015/0320556 or U.S. Patent Application Publication No. 2018/036530, the contents of which are incorporated by reference in its entirety.

The leaflets **40** can be secured to one another at their adjacent sides to form commissures **122** of the leaflet structure **(****FIG. 20****).** A plurality of flexible connectors **124** (one of which is shown in **FIG. 16****)** can be used to interconnect pairs of adjacent sides of the leaflets and to mount the leaflets to the commissure window frame portions **30 (****FIG. 5****).** **FIG. 16** shows the adjacent sides of two leaflets **40** interconnected by a flexible connector **124.** Three leaflets **40** can be secured to each other side-to-side using three flexible connectors **124,** as shown in **FIG. 17****.** Additional information regarding connecting the leaflets to each other, as well as connecting the leaflets to the frame, can be found, for example, in U.S. Patent Application Publication No. 2012/0123529, which is incorporated by reference herein in its entirety.

As noted above, the inner skirt **16** can be used to assist in suturing the leaflet structure **14** to the frame. The inner skirt **16** can have an undulating temporary marking suture to guide the attachment of the lower edges of each leaflet **40.** The inner skirt **16** itself can be sutured to the struts of frame **12** using sutures **70,** as noted above, before securing the leaflet structure **14** to the skirt **16.** The struts that intersect the marking suture desirably are not attached to the inner skirt **16.** This allows the inner skirt **16** to be more pliable in the areas not secured to the frame and minimizes stress concentrations along the suture line that secures the lower edges of the leaflets to the skirt. As noted above, when the skirt is secured to the frame, the fibers **78, 80** of the skirt (see **FIG. 12****)** generally align with the angled struts of the frame to promote uniform crimping and expansion of the frame.

**FIG. 18** shows one specific approach for securing the commissure portions **122** of the leaflet structure **14** to the commissure window frame portions **30** of the frame. The flexible connector **124 (****FIG. 17****)** secures two adjacent sides of two leaflets that are folded widthwise, and the upper tab portions **112** are folded downwardly against the flexible connector. Each upper tab portion **112** is creased lengthwise (vertically) to assume an L-shape having a first portion **142** folded against the surface of the leaflet and a second portion **144** folded against the connector **124.** The second portion **144** can then be sutured to the connector **124** along a suture line **146.** Next, the commissure tab assembly is inserted through the commissure window **20** of a corresponding window frame portion **30,** and the folds outside of the window frame portion **30** can be sutured to portions **144.**

**FIG. 18** also shows that the folded down upper tab portions **112** can form a double layer of leaflet material at the commissures. The first portions **142** of the upper tab portions **112** are positioned flat against layers of the two leaflets **40** forming the commissures, such that each commissure comprises four layers of leaflet material just inside of the window frames **30.** This four-layered portion of the commissures can be more resistant to bending or articulating than the portion of the leaflets **40** just radially inward from the relatively more-rigid four-layered portion. This causes the leaflets **40** to articulate primarily at inner edges **143** of the folded-down first portions **142** in response to blood flowing through the prosthetic valve during operation within the body, as opposed to articulating about or proximal to the axial struts of the window frames **30.** Because the leaflets articulate at a location spaced radially inwardly from the window frames **30,** the leaflets can avoid contact with and damage from the frame. However, under high forces, the four layered portions of the commissures can splay apart about a longitudinal axis adjacent to the window frame **30,** with each first portion **142** folding out against the respective second portion **144.** For example, this can occur when the prosthetic valve **10** is compressed and mounted onto a delivery shaft, allowing for a smaller crimped diameter. The four-layered portion of the commissures can also splay apart about the longitudinal axis when the balloon catheter is inflated during expansion of the prosthetic valve, which can relieve some of the pressure on the commissures caused by the balloon, reducing potential damage to the commissures during expansion.

After all three commissure tab assemblies are secured to respective window frame portions **30,** the lower edges of the leaflets **40** between the commissure tab assemblies can be sutured to the inner skirt **16.** For example, as shown in **FIG. 19****,** each leaflet **40** can be sutured to the inner skirt **16** along suture line **154** using, for example, Ethibond Excel^{®} PET thread. The sutures can be in-and-out sutures extending through each leaflet **40,** the inner skirt **16,** and each reinforcing strip **72.** Each leaflet **40** and respective reinforcing strip **72** can be sewn separately to the inner skirt **16.** In this manner, the lower edges of the leaflets are secured to frame **12** via the inner skirt **16.** As shown in **FIG. 19****,** the leaflets can be further secured to the skirt with blanket sutures **156** that extend through each reinforcing strip **72,** leaflet **40,** and the inner skirt **16** while looping around the edges of the reinforcing strips **72** and leaflets **40.** The blanket sutures **156** can be formed from PTFE suture material. **FIG. 20** shows a side view of the frame **12,** leaflet structure **14** and the inner skirt **16** after securing the leaflet structure **14** and the inner skirt **16** to the frame **12,** and the leaflet structure **14** to the inner skirt **16.**

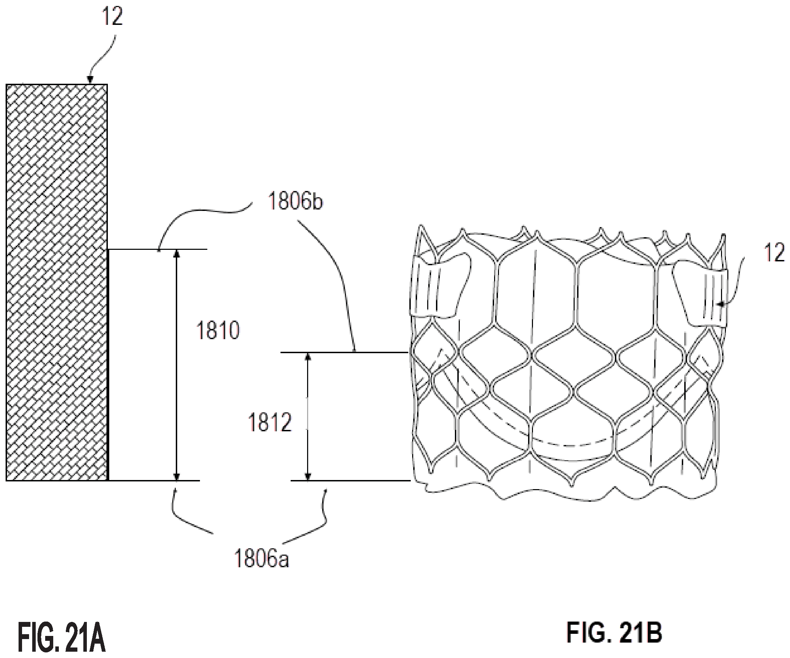

In aspects disclosed herein, the first portion has a first length extending between the proximal end and the distal end in the radially compressed configuration of the annular frame and a second length extending between the proximal end and the distal end of the first portion in the radially expanded configuration of the annular frame. These configurations are schematically shown in **FIG. 21****.**

Annular frame **12** is shown in the radially compressed configuration **(****FIG. 21A****),** where the first portion **1806** has a first length between the proximal end **1806a** and the distal end **1806b.** In **FIG. 21B****,** the annular frame **12** is shown in the radially expanded configuration where the first portion **1806** has a second length between the proximal end **1806a** and the distal end **1806b.**

In the disclosed herein aspects, the outer sealing member has a length that is substantially identical to the first length of the first portion. Thus, when the valve is in the radially compressed configuration, the outer sealing member is straightened without a substantial tension and snuggly encompasses the outer surface of the annular frame providing a good crimp profile. It is understood, however, that in some aspects, since the outer sealing member can be assembled on an expanded frame as the frame gets longer in length (radially compressed), a certain tension can be introduced along the length of the outer sealing member. However, it is further understood that such tension is lower than in any other reference device where the outer member has a length shorter than the length of the first portion.

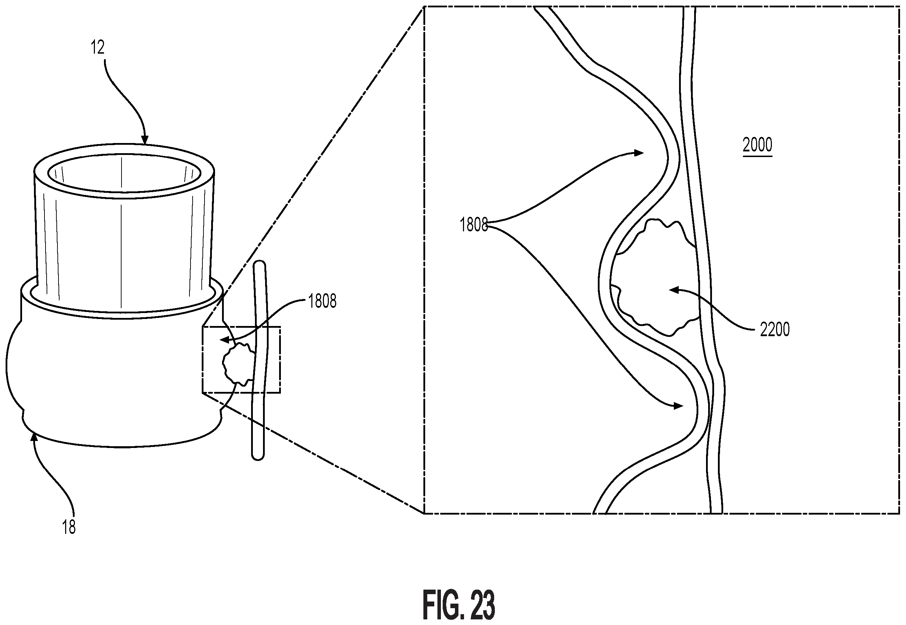

In still further aspects, when the annular frame is in the radially expanded configuration, the outer sealing member is configured to outwardly bulge as its length shortens together with the length of the annular frame. The formed bulge is then configured to fill any gaps that can be present between the prosthetic valve and the native valve. An exemplary placement of the valve in the subject's anatomy is shown in **FIG. 23****.** The valve in outer sealing member **18** forming a bulge **1808** in the radially expanded configuration of the frame can form a seal surrounding calcified aortic annuli **2200** within the vascular system **2000.**

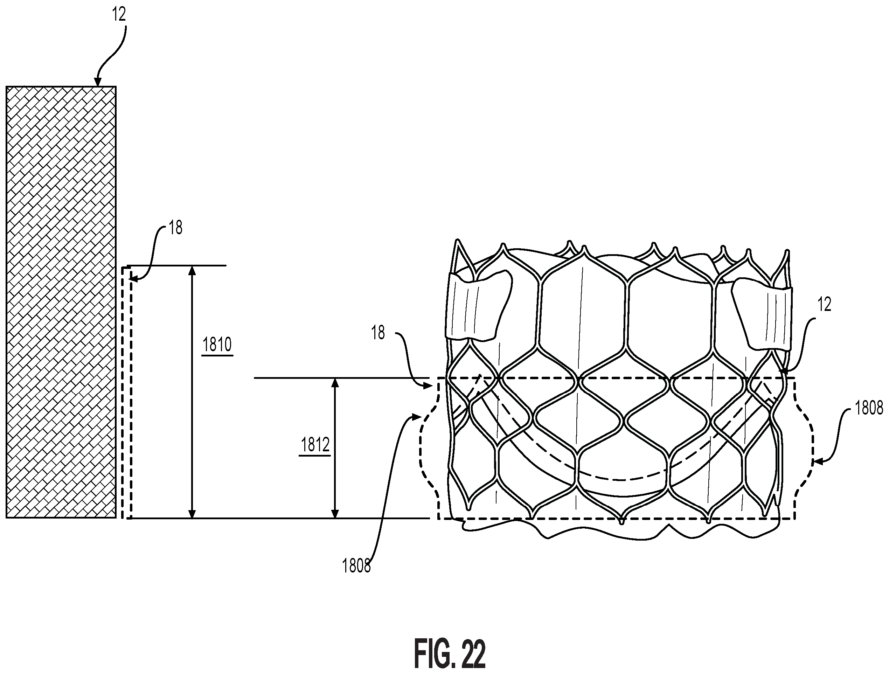

Exemplary schematic of the outer sealing member assembled on the annular frame in various configurations is also shown in **FIG. 22****.**

A right portion of **FIG. 22****,** shows the frame **12** that is in a crimped (radially compressed) configuration having the straightened outer sealing member **18** mounted on the first portion of frame **1806.** A left portion of **FIG. 22** depicts the annular frame **12** in the radially expanded configuration, where the outer sealing member **18** forms a bulge **1808.** A change in the length of the outer sealing member and the first portion of the annular frame can be observed when the annular frame changes from compressed to the expanded configuration.

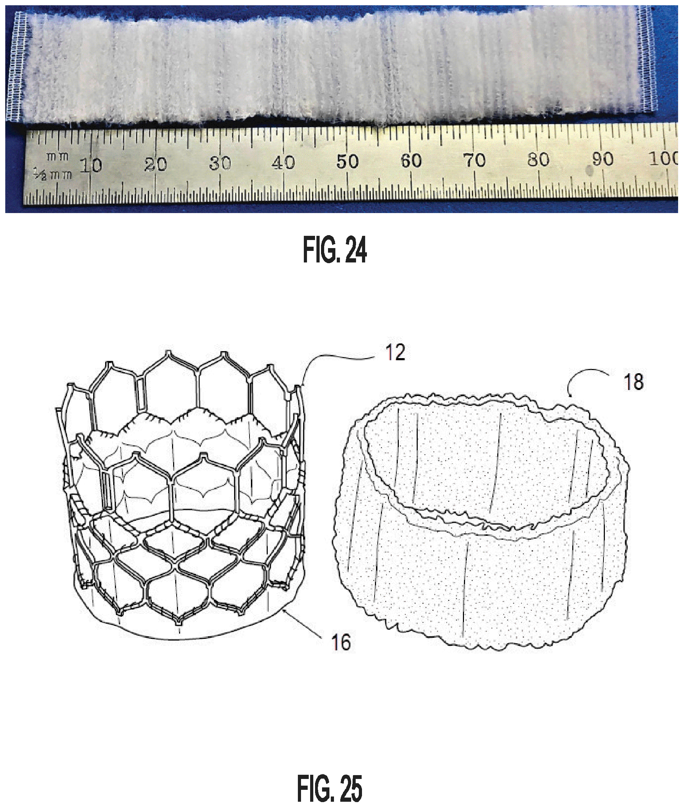

In still further aspects, the outer sealing member has a width substantially identical to a circumference of the annular frame in the expanded configuration. It is understood that the assembly of the outer sealing member and the annular frame is performed in the expanded configuration. The outer sealing member is wrapped around the annular frame, so its width is substantially identical to the circumference of the annular frame in the expanded configuration. Exemplary outer skirt is shown in **FIGS. 24** and **25. FIG. 24** shows an exemplary outer skirt as prepared prior to attaching it to the frame. It is understood that the **length** of the configuration can be obtained by either knitting or weaving the fabric to the desired height, or it can be obtained by making the fabric of any length and laser cutting or ultrasonic cutting to the desired length

**FIG. 25** shows an annular frame **12** with the mounted inner skirt **16** next to the outer skirt **18,** wherein the outer skirt is made to have a width substantially identical to a circumference of the annular frame.

The outer sealing member of the present disclosure can comprise a cloth having a mesh layer and a pile layer. In such aspects, the mesh layer has an inner surface and an outer surface. The inner surface of the mesh layer is substantially free of the pile yarns, and at least a portion of the inner surface of the mesh layer is in substantial contact with at least a portion of the outer surface of the annular frame. It is understood that the pile-free surface of the cloth can be achieved by utilizing the same or different knit or weave pattern. In still further aspects, the pile layer comprises a plurality of pile yarns that extend outwardly from at least a portion of the inner surface of the mesh layer.

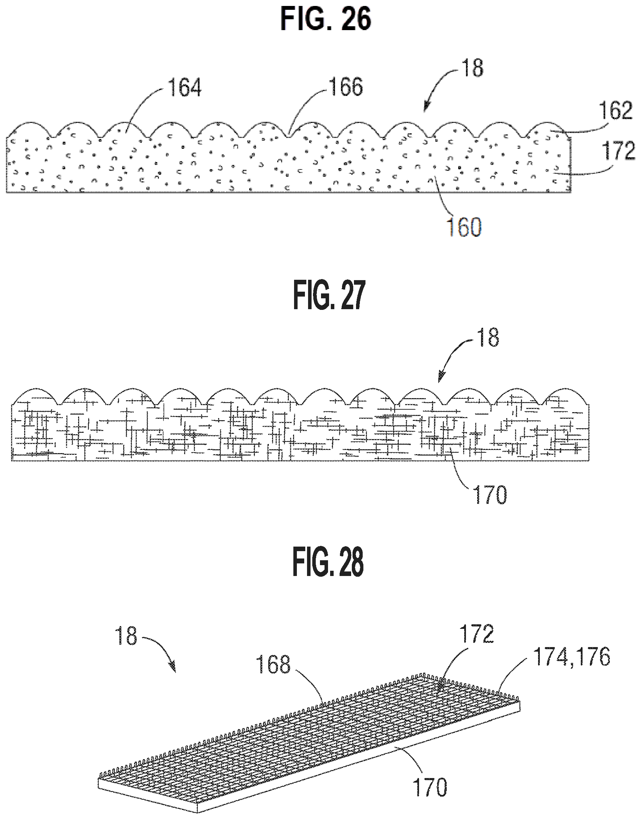

The exemplary schematic and the pictures of the cloth structure are shown in **FIGS. 26-28** and **38-41.** FIG. 26 is a flattened view of the outer skirt 18 prior to its attachment to frame 12, schematically showing the pile layer of the outer skirt. FIG. 27 is a flattened view of the outer skirt 18 prior to its attachment to frame 12, showing the mesh layer of the outer skirt. FIG. 28 is a perspective view of the outer skirt prior to its attachment to frame 12.

It is understood that the outer skirt **18** can be laser cut or ultrasonic cut or otherwise formed from strong, durable synthetic or natural materials configured to restrict and/or prevent blood flow therethrough. The outer skirt **18** can comprise a proximal (inflow or upstream) end **160** and a distal (outflow or downstream) end **162.** In certain aspects and as shown in **FIG. 24** both distal and proximal ends of the outer skirt can be substantially straight. In yet other exemplary and unlimiting aspects, and as schematically shown in **FIG. 26** and **27****,** the proximal end **160** can be substantially straight while the distal end **162** can define a plurality of alternating projections **164** and notches **166,** or castellations, that generally follow the shape of a row of struts of the frame. In yet still other exemplary aspects, the proximal and distal ends **160, 162** can have other shapes. For example, in one implementation, the proximal end **160** can be formed with a plurality of projections generally conforming to the shape of a row of struts of frame **12,** while the distal end **162** can be straight.

In still further aspects, the mesh layer has a first height extending axially along the frame, and the pile layer can comprise a second height extending axially along the frame, wherein the first height is greater than the second height. In certain aspects, a portion of the outer surface of the mesh layer at the proximal end of the outer sealing member is substantially free of the pile yarn. While in other, a portion of the outer surface of the mesh layer at the distal end of the outer sealing member is substantially free of the pile yarn. As shown in detail below, the portions of the mesh layer on the proximal end and/or distal end can be used to couple the outer skirt to the frame and/or inner skirt. It is understood that the pile-free surface of the cloth may be achieved by utilizing the same or different knit or weave pattern.

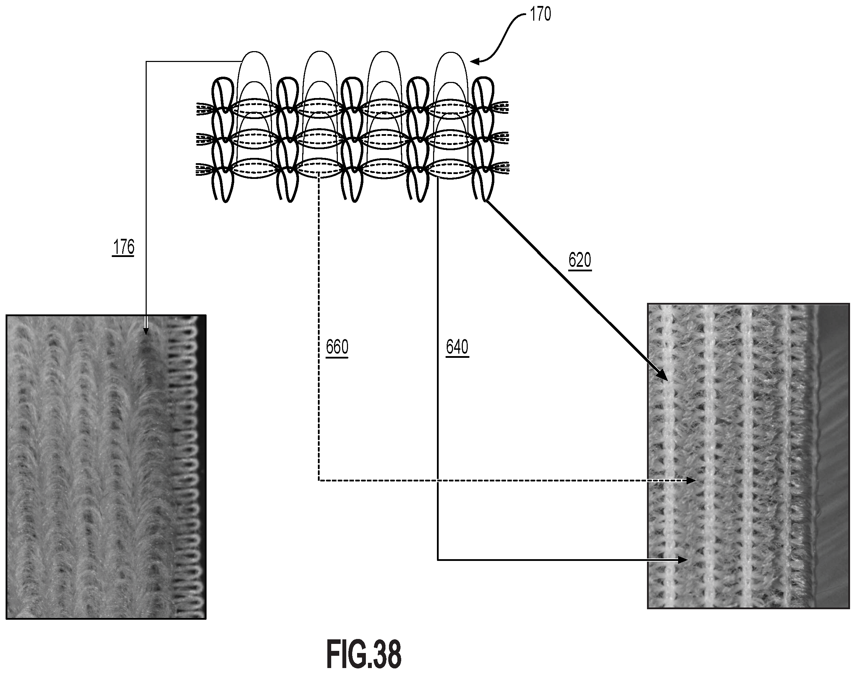

The schematic view of the fabric structure is shown in **FIG.** 38. The mesh layer **170** can comprise a plurality of warp and weft yarns such as **620, 640,** and **660.** The pile layer comprises a plurality of pile yarns **174** that extend outwardly from the mesh layer 170.

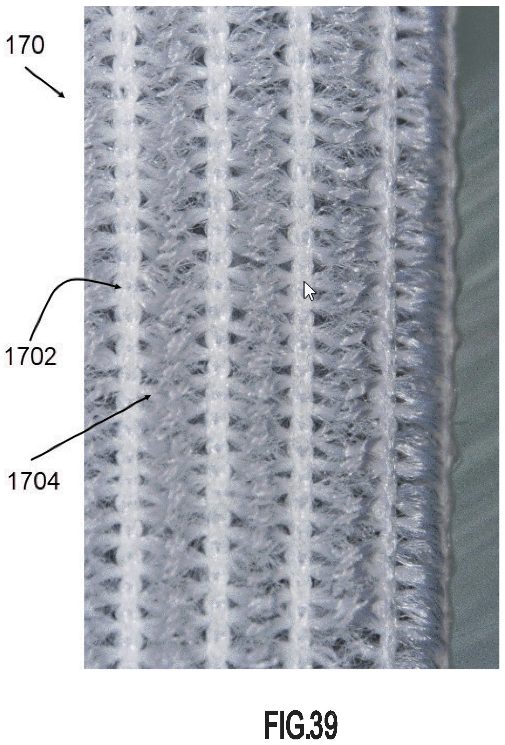

As can be seen in **FIG.** 39, the mesh layer **170** comprises a plurality of wales **1702** extending axially across the length of the outer sealing layer and a plurality of courses **1704** extending circumferentially along the width of the outer sealing layer.

In certain aspects, the mesh layer **170** can have a wales density from about 10 to about 50 wales per inch, including exemplary values of about 11 wales per inch, about 12 wales per inch, about 13 wales per inch, about 14 wales per inch, about 15 wales per inch, about 16 wales per inch, about 17 wales per inch, about 18 wales per inch, about 19 wales per inch, about 20 wales per inch, about 21 wales per inch, about 22 wales per inch, about 23 wales per inch, about 24 wales per inch, about 25 wales per inch, about 26 wales per inch, about 27 wales per inch, about 28 wales per inch, about 29 wales per inch, about 30 wales per inch, about 31 wales per inch, about 32 wales per inch, about 33 wales per inch, about 34 wales per inch, about 35 wales per inch, about 36 wales per inch, about 37 wales per inch, about 38 wales per inch, about 39 wales per inch, about 40 wales per inch, about 41 wales per inch, about 42 wales per inch, about 43 wales per inch, about 44 wales per inch, about 45 wales per inch, about 46 wales per inch, about 47 wales per inch, about 48 wales per inch, and about 49 wales per inch.. It is understood that any number between any two of the foregoing numbers of wales can be present in the disclosed herein mesh layer.

In other aspects, the mesh layer **170** can have a courses density from about 25 to about 85 courses per inch, including exemplary values of about 30 courses per inch, about 35 courses per inch, about 40 courses per inch, about 45 courses per inch, about 50 courses per inch, about 55 courses per inch, about 60 courses per inch, about 65 courses per inch, about 70 courses per inch, about 75 courses per inch, and about 80 courses per inch. It is understood that any number between any two of the foregoing numbers of courses can be present in the disclosed herein mesh layer.

In still further aspects, the plurality of wales **1702** can comprise any known in the art warp yarns. In some aspects, the warp yarn can be fully-drawn, spin drawn, or low- or not-twisted. In yet other aspects, any combinations of the disclosed herein or other known yarns can be utilized.

In still further aspects, the warp yarn can have any size suitable for the desired application. For example and without limitations, the warp yarn can have a size from about 10 denier to about 40 denier, including exemplary values of about 12 denier, about 15 denier, about 18 denier, about 20 denier, about 22 denier, about 25 denier, about 28 denier, about 30 denier, about 32 denier, about 35 denier, and about 38 denier. It is understood that the yarn can have any denier values that fall between any two foregoing values.

In still further aspects, the warp yarn can have any filament count. For example and without limitations, the warp yarn used herein can have a filament count from about 6 to about 56, including exemplary values of about 7, about 8, about 10, about 12, about 15, about 18, about 20, about 22, about 25, about 28, about 30, about 32, about 35, about 38, about 40, about 42, about 45, about 48, about 55, about 52, and about 55. It is understood that the yarn can have any filament count that falls between any two foregoing values.

In still further aspects, the warp yarn can have a size from about 10 denier to about 40 denier, including exemplary values of about 12 denier, about 15 denier, about 18 denier, about 20 denier, about 22 denier, about 25 denier, about 28 denier, about 30 denier, about 32 denier, about 35 denier, and about 38 denier, and a filament count from about 6 to about 56, including exemplary values of about 7, about 8, about 10, about 12, about 15, about 18, about 20, about 22, about 25, about 28, about 30, about 32, about 35, about 38, about 40, about 42, about 45, about 48, about 55, about 52, and about 55.

In still further aspects, the warp yarn can have tenacity from about 30 cN/tex to about 400 cN/tex, including exemplary values of about 32 cN/tex, about 35 cN/tex, about 38 cN/tex, about 40 cN/tex, about 42 cN/tex, about 45 cN/tex, about 48 cN/tex, about 50 cN/tex, about 52 cN/tex, about 55 cN/tex, about 58 cN/tex, about 60 cN/tex, about 62 cN/tex, about 65 cN/tex, about 68 cN/tex, about 70 cN/tex, about 72 cN/tex, about 75 cN/tex, about 80 cN/tex, d about 82 cN/tex, about 85 cN/tex, about 90 cN/tex, about 95 cN/tex, about 100 cN/tex, about 110 cN/tex, about 150 cN/tex, about 180 cN/tex, about 200 cN/tex, about 220 cN/tex, about 250 cN/tex, about 280 cN/tex, about 300 cN/tex, about 320 cN/tex, about 350 cN/tex, and about 380 cN/tex . It is understood that the yarn can have any tenacity value between any two foregoing values.

In still further aspects, the plurality of courses **1704** can be formed with a weft yarn, wherein the weft yarn can comprise any yarn suitable for the desired application. In some aspects, the weft yarn can comprise a multifilament configuration comprising a twisted yarn, a flat yarn, a textured yarn, or any combination thereof. In still further aspects, the plurality of courses can be formed with a single yarn, a plurality of the same yarn, or a combination of different yarns. In other aspects, the plurality of courses is formed with a weft yarn, wherein the weft yarn is a monofilament yarn. In still further aspects, the plurality of courses is formed with a weft yarn, wherein the weft yarn comprises a composite construction in the form of a covered yarn. For example, and without limitations, the weft yarn can be a combination of the twisted yarn or the flat yarn with the textured yarn.

In certain aspects, the twisted yarn and/or flat yarn can have any desirable size. In some exemplary aspects, the twisted yarn and/or flat yarn can have a size from about 10 denier to about 40 denier, including exemplary values of about 12 denier, about 15 denier, about 18 denier, about 20 denier, about 22 denier, about 25 denier, about 28 denier, about 30 denier, about 32 denier, about 35 denier, and about 38 denier. It is understood that the twisted yarn and/or flat yarn can have any denier values that fall between any two foregoing values.

In yet other aspects, the textured yarn can have any desired size. In some exemplary aspects, the textured yarn has a size of about 20 denier to about 160 denier, including exemplary values of about 30 denier, about 40 denier, about 50 denier, about 60 denier, about 70 denier, about 80 denier, about 90 denier, about 100 denier, about 120 denier, about 130 denier, about 140 denier, and about 150 denier. It is understood that the textured yarn can have any denier values that fall between any two foregoing values.

In still further aspects, the weft yarn can be formed by a combination of the twisted yarn and/or flat yarn with the textured yarn, wherein the twisted yarn and/or flat yarn can have a size from about 10 denier to about 40 denier, including exemplary values of about 12 denier, about 15 denier, about 18 denier, about 20 denier, about 22 denier, about 25 denier, about 28 denier, about 30 denier, about 32 denier, about 35 denier, and about 38 denier, and wherein the textured yarn has a size from about 20 denier to about 160 denier, including exemplary values of about 30 denier, about 40 denier, about 50 denier, about 60 denier, about 70 denier, about 80 denier, about 90 denier, about 100 denier, about 120 denier, about 130 denier, about 140 denier, and about 150 denier.

In yet further aspects, any of the yarns present in the weft yarn can have a filament count from about 10 to about 200, including exemplary values of about 20, about 30, about 40, about 50, about 60, about 70, about 80, about 90, about 100, about 110, about 120, about 130, about 140, about 150, about 160, about 170, about 180, and about 190. It is understood that any of the yarns present in the weft yarn can have a filament count value between any two foregoing values.

Such exemplary aspects are shown in FIG. 38, wherein the flat yarn 640 is combined with the textured yarn 660 to form the plurality of courses. In such exemplary aspects, the textured yarn can fill gaps in the mesh layer. In still further exemplary and unlimiting aspects, the textured can be used to make a part or all of the pile surface.

In still further aspects, the warp yarn and/or the weft yarn can comprise biocompatible thermoplastic polymers. Yet, in other aspects, the warp yarn and/or the weft yarn can comprise biocompatible non-resorbable polymers. For example, the warp yarn and/or the weft yarn can comprise polyester, co-polyester, polyamide, polyolefin, polyaryletherketones, aromatic polymers, polyurethane, or any combination thereof. Yet, in some other examples, the warp yarn and/or the weft yarn can comprise a polyethylene terephthalate (PET), polytetrafluoroethylene (PTFE), Nylon, UHMWPE, PEEK, Liquid Crystalline Polymer, thermoplastic polyurethane (TPU), or a combination thereof. Still further, any other suitable natural or synthetic fibers or any combination thereof can be used.

It is understood that the mesh layer can be a knit or woven fabric. In still further aspects, the mesh layer is velour. In some aspects, where the mesh layer is a knit fabric, it can be a crochet knit or a warp-knit fabric. However, it is understood that any known in the art knitted fabrics can be used as a mesh layer disclosed herein.

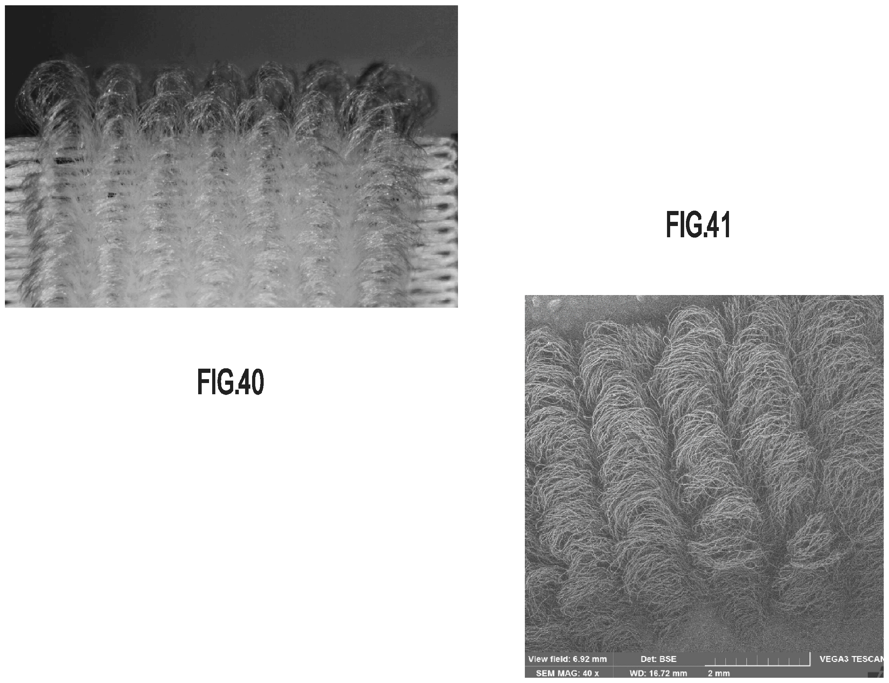

In certain aspects, the pile yarns can be arranged to form a looped pile **176** (for example, shown in **FIG.** 38). Some additional looped pile is shown in **FIGS. 40-41****,** wherein **FIG.41** shows an SEM image of the pile layer.

In some aspects, the pile yarn can also be cut to form a cut pile.

In some aspects, the pile yarn can comprise a flat or textured yarn. In yet further aspects, the pile yarn can comprise a combination of the flat and textured yarn.

In some aspects, the pile yarn can have a size from about 20 denier to about 80 denier, including exemplary values of about 25 denier, about 30 denier, about 35 denier, about 40 denier, about 45 denier, about 50 denier, about 55 denier, about 60 denier, about 65 denier, about 70 denier, and about 75 denier. It is understood that the pile yarn can have any denier values that fall between any two foregoing values.

In still other aspects, the pile yarn can have a filament count from about 10 to about 100, including exemplary values of about 15, about 20, about 25, about 30, about 35, about 40, about 45, about 50, about 55, about 60, about 65, about 70, about 75, about 80, about 85, about 90, and about 95. It is understood that pile yarn can have a filament count value between any two foregoing values.

In still further aspects, the pile yarn can comprise biocompatible thermoplastic polymers. Yet, in other aspects, the pile yarn can comprise biocompatible non-resorbable polymers. For example, the warp yarn and/or the weft yarn can comprise polyester, co-polyester, polyamide, polyolefin, polyaryletherketones, aromatic polymers, polyurethane, or any combination thereof. Yet, in some other examples, the warp yarn and/or the weft yarn can comprise a polyethylene terephthalate (PET), polytetrafluoroethylene (PTFE), Nylon, UHMWPE, PEEK, Liquid Crystalline Polymer, thermoplastic polyurethane (TPU), or a combination thereof. Still further, any other suitable natural or synthetic fibers or any combination thereof can be used.

In still further aspects, the pile layer can include velour, velvet, velveteen, corduroy, terrycloth, fleece, etc.

A schematic representation of the outer sealing member can also be seen in **FIG. 28****.** In particular aspects, the outer skirt **18** can comprise at least one soft, plush surface **168** oriented radially outward so as to cushion and seal against native tissues surrounding the prosthetic valve. In certain examples, the outer skirt **18** can be made from any of a variety of woven, knitted, or crocheted fabrics, wherein the surface **168** is the surface of a plush nap or pile of the fabric. Exemplary fabrics having a pile include velour, velvet, velveteen, corduroy, terrycloth, fleece, etc. As best shown in **FIG. 28****,** the outer skirt can have a mesh layer **170** as described above, from which a pile layer **172,** as also described above, extends.

The pile layer **172** can comprise pile yarns **174** woven or knitted into loops. In certain configurations, the pile yarns **174** can be the warp yarns or the weft yarns of the mesh layer **170** woven or knitted to form the loops. The pile yarns **174** can also be separate yarns incorporated into the mesh layer, depending upon the characteristics desired. In certain aspects, and as disclosed above, the loops can be cut such that the pile layer **172** is a cut pile in the manner of, for example, a velour fabric. In other aspects, the loops can be left intact to form a looped pile in the manner of, for example, terrycloth.

The height of the pile yarns **174** (e.g., the loops **176)** can be the same for all pile yarns across the entire extent of the outer skirt so as to provide an outer skirt having a constant thickness. This can provide a uniform crimping profile of the valve from the inflow to the outflow. In alternative aspects, the height of the pile yarns **174** can vary along the circumference of the outer skirt so as to vary the thickness of the outer skirt along its circumference. In certain aspects, it is preferable that the height of the pile along the length of the valve is not varied. Also, while the height of the pile yarn along the circumference can vary, it may not be preferable as it can leave gaps in the axial direction of the valve, which is the direction of the flow of blood. As a result, the sealing of the valve can be affected.

The pile layer **172** has a much greater surface area than similarly sized skirts formed from flat or woven materials and therefore can enhance tissue ingrowth compared to known skirts. Promoting tissue growth into the pile layer **172** can decrease perivalvular leakage, increase retention of the valve at the implant site and contribute to long-term stability of the valve. In some configurations, the surface area of the pile yarns **174** can be further increased by using textured yarns having an increased surface area due to, for example, a wavy or undulating structure. In configurations such as the looped pile aspect of **FIG. 40****,** the loop structure and the increased surface area provided by the textured yarn of the loops can allow the loops to act as a scaffold for tissue growth into and around the loops of the pile.

As disclosed in detail above, the pile yarns in the pile layer can have a height that is substantially the same along the length and/or width of the outer sealing member (or the outer skirt). This exemplary aspect is shown in **FIG. 3****.** As discussed above, **FIG. 3** shows an exemplary attachment of the outer skirt **18** to the radially compressed annular frame. In this exemplary aspect, the height of the loops of the pile layer **172** is constant across the entire extent of the outer skirt such that the outer skirt **18** has a constant thickness, except along the distal and proximal end of the outer sealing member, which can be free of loops (pile yarn) to facilitate attachment of the outer skirt to the frame and/or the inner skirt **16.**

The "height" of the loops is measured in the radial direction when the skirt is mounted on the radially compressed frame. The skirt is mounted on an expanded frame, and the height of loops or the outer sealing member is measured before being assembled onto the frame. In another aspect, the pile yarn can have a height that varies along the length and/or width of the outer sealing member.

In lieu of or in addition to having loops that vary in height along the height of the skirt, and as discussed above, the height of the loops **176** (and therefore the thickness of the outer skirt) can vary along the circumference of the outer skirt. For example, the height of the loops can be increased along circumferential sections of the outer skirt where larger gaps might be expected between the outer skirt and the native annulus, such as circumferential sections of the skirt that are aligned with the commissures of the native valve. It is understood that in some aspects, it is preferable to have a height of the loops substantially identical along the length and the circumference of the skirt.

The outer skirt aspects described herein can also contribute to improved compressibility and shape memory properties of the outer skirt over known valve coverings and skirts. For example, the pile layer **172** can be compliant such that it compresses under load (e.g., when in contact with tissue, other implants, or the like) and returns to its original size and shape when the load is relieved. This can help to improve sealing between the outer skirt and the tissue of the native annulus or a surrounding support structure in which the prosthetic valve is deployed. Aspects of an implantable support structure that is adapted to receive a prosthetic valve and retain it within the native mitral valve are disclosed in co-pending Application No. 62/449,320, filed Jan. 23, 2017, and application Ser. No. 15/876,053, filed Jan. 19, 2018, which are incorporated herein by reference. The compressibility provided by the pile layer **172** of the outer skirt **18** is also beneficial in reducing the crimp profile of the valve. Additionally, the outer skirt **18** can prevent the leaflets **40** or portions thereof from extending through spaces between the struts of frame 12 as the prosthetic valve is crimped, thereby protecting against damage to the leaflets due to pinching of the leaflets between struts.

In still further aspects, the outer sealing member can have an uncompressed thickness of about 0.6 mm to about 2.5 mm, including exemplary values of about 0.7 mm, about 0.8 mm, about 0.9 mm, about 1.0 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, about 2.2 mm, about 2.3 mm, and about 2.4 mm. It is understood that the outer sealing member can have any uncompressed thickness value between any two disclosed above values.

In still further aspects, the outer sealing member can have a compressed thickness of about 0.4 mm to about 1 mm, including exemplary values of about 0.5 mm, about 0.6 mm, about 0.7 mm, about 0.8 mm, and about 0.9 mm, wherein the outer sealing member is compressed at 23.4+/-0.7IPa pressure.

In certain aspects, the distal and the proximal end of the outer sealing member can comprise at least two threads of the weft yarn. In such exemplary and unlimiting aspects, the presence of the two threads of the weft yarn improves the durability of the outer skirt and the convenience of coupling it to the annular frame and/or inner skirt.

In alternative aspects, the outer skirt **18** be made of a non-woven fabric such as felt or fibers such as non-woven cotton fibers. The outer skirt **18** can also be made of porous or spongey materials such as, for example, any of a variety of compliant polymeric foam materials or woven fabrics, such as woven PET.

Some additional examples of outer skirts can be found in U.S. Patent Application Publication No. 2019/0365530, the content of which is incorporated herein in its whole entirety.

Various techniques and configurations can be used to secure the outer skirt **18** to frame **12** and/or the inner skirt **16.** In certain aspects, the outer sealing member **18** can be coupled to the proximal end **1806a** and the distal end **1806b** of the first portion by a fastener. In still further aspects, a fastener can be used to couple the outer skirt **18** to the inner skirt **16.** It is understood that the fasteners can comprise any fasteners known in the art. For example, and without limitations, the fasteners can comprise sutures, pins, rivets, ultrasonic welding, laser welding, adhesive bonding, or any combination thereof.

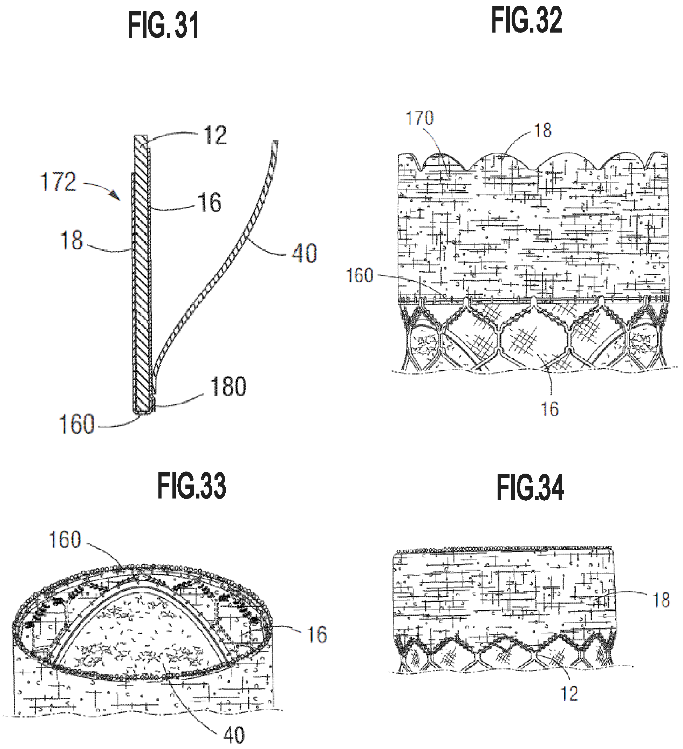

**FIG.** 3 shows an exemplary attachment of the outer skirt **18** to the frame **12** when the frame is in the radially compressed configuration, and the outer skirt **18** is straightened along the outer surface of the first portion of the compressed annular frame. In such exemplary aspect, a lower edge portion **180** of the inner skirt **16** can be wrapped around the inflow end **15** of the frame **12,** and the lower edge portion **160** of the outer skirt **18** can be attached to the lower edge portion **180** of the inner skirt **16** and/or the frame **12,** such as with a fastener that comprises one or more sutures or stitches **182** (as best shown in **FIG. 2****)** and/or an adhesive. In lieu of or in addition to sutures, the outer skirt **18** can be attached to the inner skirt **16,** for example, by ultrasonic welding. In the exemplary and unlimiting illustrated aspect, the lower edge portion **160** of the outer skirt **18** can be free of loops (or in other words, it can be attached to a portion that a substantially free of the pile yarn), and the lower edge portion **180** of the inner skirt **16** can overlap and can be secured to the mesh layer **170** of the outer skirt **18.** In other aspects, the proximal edge portion **180** of the inner skirt **16** can extend over one or more rows of the loops **176** of the pile layer **172** (see **FIG. 32****),** as further described below. In other aspects, the lower edge portion **180** of the inner skirt **16** can be wrapped around the inflow end **15** of the annular frame and extend between the outer surface of the frame and the outer skirt **18** (i.e., the outer skirt **18** is radially outward of the lower edge portion **180** of the inner skirt 16).

The outer skirt can be attached in the distal end of the first portion to the rows of struts of the annular frame as described below. For example, as shown in **FIG. 1****,** each projection **164** of the outer skirt **18** can be attached to the third row III of struts **26 (****FIG. 5****)** of the frame **12.** The projections **164** can, for example, be wrapped over respective struts **26** of row III and secured with sutures **184.** The outer skirt **18** can be further secured to the frame **12** by suturing an intermediate portion of the outer skirt (a portion between the proximal end and distal end) to struts of the frame, such as struts **24** of the second row II of struts.

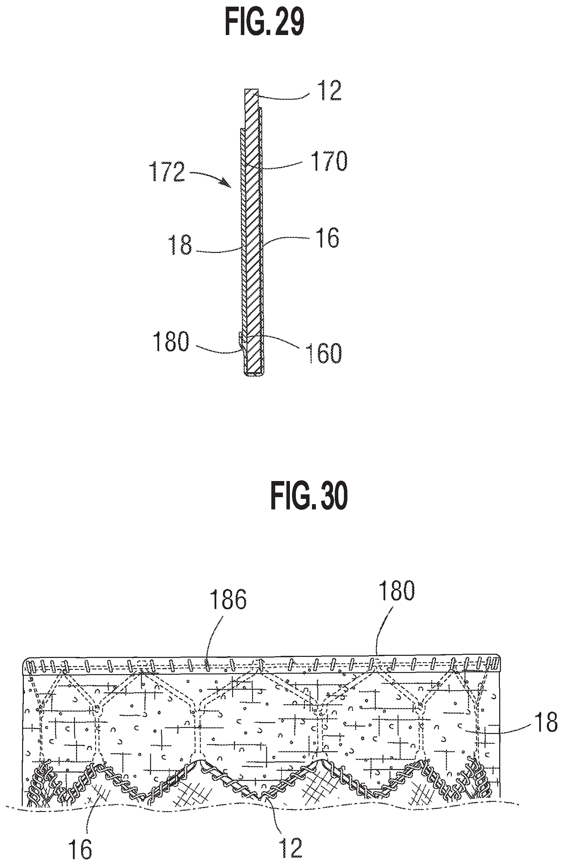

**FIGS. 29** and **30** show an alternative and unlimiting configuration for mounting the outer skirt **18** to frame 12. In this aspect, as best shown in **FIG. 29****,** (again it is understood that the configurations disclosed herein relate to the annular frame present in the compressed configuration when the outer skirt is straightened along the first portion of the annular frame), the proximal end **180** of the inner skirt **16** is wrapped around the inflow end **15** of the frame and extended over one or more rows of loops along the proximal end **160** of the outer skirt **18.** The proximal portion **180** of the inner skirt **16** can then be secured to the proximal portion **160** of the outer skirt **18,** such as with sutures or stitching **186 (****FIG. 30****),** an adhesive, and/or welding (e.g., ultrasonic welding). The stitching **186** can also extend around selected struts adjacent the inflow end **15** of the frame. In this exemplary and unlimiting aspect, the proximal portion **180** of the inner skirt can effectively to partially compress the loops of the pile layer **172,** thereby creating a tapered edge at the inflow end of the prosthetic valve. The tapered edge can reduce the insertion force required to push the prosthetic valve through an introducer sheath when being inserted into a patient's body. In one exemplary and unlimiting implementation, the stitching **186** can secure the proximal end **180** of the inner skirt **16** to the outer skirt **18** at a distance of at least 1 mm from the most proximal end of the outer skirt **18.** The distal portion **162** and the intermediate portion of the outer skirt can then be secured to the frame as previously described.

**FIGS. 31-34** show additional and unlimiting aspects referring to another configuration for mounting the outer skirt **18** to frame **12** that is in the annular compressed configuration. In this exemplary aspect, the outer skirt **18** can be initially placed in a tubular configuration with the mesh layer **170** facing outwardly. The proximal end **160** (which can be free of loops **176)** can be placed between the inner surface of the frame **12** and the proximal portion **180** of the inner skirt **16,** as depicted in **FIG. 31****.** The proximal end of the outer skirt **18** and the inner skirt **16** can be then secured to each other, such as with stitches, an adhesive, and/or welding (e.g., ultrasonic welding). In one aspect, the proximal portions of the outer skirt and the inner skirt can also be secured to each other with in-and-out stitches and locking stitches. The outer skirt **18** can be then inverted and pulled upwardly around the outer surface of the frame **12 such** that the mesh layer **170** is placed against and is in substantial contact with the outer surface of the frame and the pile layer **172** faces outwardly, as depicted in **FIG. 31****.** In this assembled configuration, the proximal end **160** of the outer skirt **18** can wrap around the inflow end **15** of the frame and is secured to the inner skirt inside of the frame. The distal end **162** and the intermediate portion of the outer skirt can then be secured to the frame as previously described.

**FIG. 34** schematically shows the outer skirt attached to the annular frame prior to the attachment to the inner skirt at the proximal end of the frame. **FIG. 40** shows the outer skirt attached to the annular frame in the expanded configuration when a bulge **1808** is formed.

The prosthetic valve **10** can be configured for and mounted on a suitable delivery apparatus for implantation in a subject. Several catheter-based delivery apparatuses are known; a non-limiting example of a suitable catheter-based delivery apparatus includes that disclosed in U.S. Patent Application Publication No. 2013/0030519, which is incorporated by reference herein in its entirety, and U.S. Patent Application Publication No. 2012/0123529.

To implant a plastically-expandable prosthetic valve **10** within a subject, the prosthetic valve **10,** including the outer skirt **18,** can be crimped on an elongated shaft of a delivery apparatus. It is understood that in crimped (compressed configuration), the outer skirt **18** is snugly fit around the circumference of the annular frame without creating a substantial tension within the outer skirt fabric. It is understood, however, that in some aspects, the outer sealing member can be assembled on an expanded frame as the frame gets longer in length (radially compressed).

The prosthetic valve, together with the delivery apparatus, can form a delivery assembly for implanting the prosthetic valve **10** in a subject's body. The shaft can comprise an inflatable balloon for expanding the prosthetic valve within the body. With the balloon deflated, the prosthetic valve **10** can then be percutaneously delivered to the desired implantation location (e.g., a native aortic valve region). Once prosthetic valve **10** is delivered to the implantation site (e.g., the native aortic valve) inside the body, the prosthetic valve 10 can be radially expanded to its functional state by inflating the balloon or equivalent expansion mechanism.

The outer skirt **18,** when the frame is in the expanded configuration, forms a bulge that can tightly seal against the surrounding native annulus forming a good, fluid-tight seal between the prosthetic valve **10** and the native annulus. The outer skirt **18,** therefore, cooperates with the inner skirt **16** to avoid perivalvular leakage after implantation of the prosthetic valve **10.** Additionally, as discussed above, the pile layer of the outer skirt further enhances perivalvular sealing by promoting tissue ingrowth with the surrounding tissue.

Alternatively, a self-expanding prosthetic valve **10** can be crimped to a radially collapsed configuration and restrained in the collapsed configuration by inserting the prosthetic valve **10,** including the outer skirt **18,** into a sheath or equivalent mechanism of a delivery catheter. The prosthetic valve **10** can then be percutaneously delivered to the desired implantation location. Once inside the body, the prosthetic valve **10** can be advanced from the delivery sheath, which allows the prosthetic valve to expand to its functional state.

### METHODS

The present disclosure also provides for a of forming an implantable prosthetic valve comprising: a) providing an annular frame having an inner surface and an outer surface wherein the frame has an inflow end and an outflow end; wherein the annular frame is compressible and expandable between a radially compressed configuration and a radially expanded configuration; b) circumferentially mounting an outer sealing member having a proximal end and a distal end around a first portion of the outer surface of the annular frame, wherein the first portion of the outer surface has a proximal end, and a distal end, wherein the proximal end of the first portion is at the inflow end of the annular frame, and wherein the first portion has a first length extending between the proximal end and the distal end in the radially compressed configuration of the annular frame and a second length extending between the proximal end and the distal end in the radially expanded configuration of the annular frame; wherein the outer sealing member has a length substantially identical to the first length of the first portion; and wherein the outer sealing member is configured to outwardly bulge when the annular frame is in the radially expanded configuration, thereby forming a seal against surrounding tissue when the prosthetic valve is implanted.

It is understood that in some aspects, the annular frame is radially expanded to the expanded configuration prior to the step of mounting. In such exemplary aspects, the outer sealing member is snugly fitted around an expanded circumference of the annular frame. It is understood, however, and as described in detail above, the outer sealing member has a length that is substantially identical to the length of the first portion when the annular frame is in a crimped or compressed configuration. Thus, when the outer skirt is mounted on the expanded frame, the outer skirt forms a budge that is configurated to seal against native anatomy when it is positioned in the subject's body.

Any of the disclosed above outer skirts can be used to form the valve. Similarly, any of the methods disclosed above of attachment of the outer skirt to the annular frame can be utilized.

In still further aspects, the methods disclosed herein can comprise a step of impregnating any of the disclosed herein textile materials with a pharmaceutically active agent depending on the desired application. In still further aspects, the methods disclosed herein can comprise a step of coating any of the disclosed herein textile materials with any known in the art materials that can provide for any additional desired properties.

Although several aspects of the invention have been disclosed in the foregoing specification, it is understood by those skilled in the art that many modifications and other aspects of the invention will come to mind to which the invention pertains, having the benefit of the teaching presented in the foregoing description and associated drawings. It is thus understood that the invention is not limited to the specific aspects disclosed hereinabove and that many modifications and other aspects are intended to be included within the scope of the appended claims. Moreover, although specific terms are employed herein, as well as in the claims which follow, they are used only in a generic and descriptive sense and not for the purposes of limiting the described invention nor the claims which follow. We, therefore, claim as our invention all that comes within the scope and spirit of these claims.

### EXEMPLARY ASPECTS

**Example 1:** An implantable prosthetic valve comprising: a) an annular frame having an inner surface and an outer surface, an inflow end and an outflow end; wherein the annular frame is compressible and expandable between a radially compressed configuration and a radially expanded configuration; and b) an outer sealing member having a proximal end and a distal end and is mounted circumferentially around a first portion of the outer surface of the annular frame, wherein the first portion of the outer surface having a proximal end and a distal end, wherein the proximal end of the first portion is at the inflow end of the annular frame, and wherein the first portion has a first length extending between the proximal end and the distal end in the radially compressed configuration of the annular frame and a second length extending between the proximal end and the distal end in the radially expanded configuration of the annular frame, wherein the outer sealing member has a length substantially identical to the first length of the first portion; and wherein the outer sealing member is configured to outwardly bulge when the annular frame is in the radially expanded configuration, thereby forming a seal against surrounding tissue when the prosthetic valve is implanted.

**Example 2:** The implantable prosthetic valve of any examples herein, particularly example1, wherein the outer surface of the annular frame has a second portion that is free of the outer sealing member and wherein the second portion extends between the outflow end of the annular frame and the distal end of the first portion.

**Example 3:** The implantable prosthetic valve of any examples herein, particularly example 1 or 2, wherein the proximal end of the outer sealing member is coupled to the proximal end of the first portion, and the distal end of the outer sealing member is coupled to the distal end of the first portion.

**Example 4:** The implantable prosthetic valve of any examples herein, particularly example 3, wherein the outer sealing member is coupled to the proximal end and the distal end of the first portion by a fastener.

**Example 5:** The implantable prosthetic valve of any examples herein, particularly example 4, wherein the fastener comprises sutures, pins, rivets, ultrasonic welding, laser welding, adhesive bonding, or any combination thereof.

**Example 6:** The implantable prosthetic valve of any examples herein, particularly examples 1-5, wherein the outer sealing member is substantially straightened along the first portion of the outer surface of the annular frame when the annular frame is in the radially compressed configuration.

**Example 7:** The implantable prosthetic valve of any examples herein, particularly examples 1-6, wherein the outer sealing member has a width substantially identical to a circumference of the annular frame in the expanded configuration.

**Example 8:** The implantable prosthetic valve of any examples herein, particularly examples 1-7, wherein the outer sealing member comprises a mesh layer and a pile layer, wherein the pile layer comprises a plurality of pile yarns extending outwardly from at least a portion of an outer surface of the mesh layer, and wherein an inner surface of the mesh layer is substantially free of the pile yarns and wherein at least a portion of the inner surface of the mesh layer is in substantial contact with at least a portion of the outer surface of the annular frame.

**Example 9:** The implantable prosthetic valve of any examples herein, particularly example 8, wherein the mesh layer has a first height extending axially along the frame, and the pile layer comprises a second height extending axially along the frame, wherein the first height is greater than the second height.

**Example 10:** The implantable prosthetic valve of any examples herein, particularly example 8 or 9, wherein a portion of the outer surface of the mesh layer at the proximal end of the outer sealing member is substantially free of the pile yarn.

**Example 11:** The implantable prosthetic valve of any examples herein, particularly examples 8-10, wherein a portion of the outer surface of the mesh layer at the distal end of the outer sealing member is substantially free of the pile yarn.

**Example 12:** The implantable prosthetic valve of any examples herein, particularly example 10 or 11, wherein the portion of the outer surface of the mesh layer at the proximal end of the outer sealing member that is substantially free of the pile yarn is sutured to the proximal end of the first portion of the annular frame.

**Example 13:** The implantable prosthetic valve of any examples herein, particularly example 11 or 12, wherein the portion of the outer surface of the mesh layer at the distal end of the outer sealing member that is substantially free of the pile yarn is sutured to the distal end of the first portion of the annular frame.

**Example 14:** The implantable prosthetic valve of any examples herein, particularly examples 8-13, wherein the mesh layer comprises a plurality of warp and weft yarns and comprises a plurality of wales extending axially across the length of the outer sealing layer and a plurality of courses extending circumferentially along the width of the outer sealing layer.

**Example 15:** The implantable prosthetic valve of any examples herein, particularly example 14, wherein the mesh layer has a wales density from about 10 to about 50 wales per inch.

**Example 16:** The implantable prosthetic valve of any examples herein, particularly example 14 or 15, wherein the mesh layer has a courses density from about 25 to about 85 courses per inch.

**Example 17:** The implantable prosthetic valve of any examples herein, particularly examples 14-16, wherein the plurality of wales comprises a warp yarn, wherein the warp yarn is fully-drawn, or spin-drawn or low or not twisted.

**Example 18:** The implantable prosthetic valve of any examples herein, particularly example 17, wherein the warp yarn has a size from about 10 denier to about 40 denier and a filament count from about 6 to about 56.

**Example 19:** The implantable prosthetic valve of any examples herein, particularly example 17 or 18, wherein the warp yarn has a tenacity from about 30 to about 400 cN/tex.

**Example 20:** The implantable prosthetic valve of any examples herein, particularly examples 14-19, wherein the plurality of courses are formed with a weft yarn, wherein the weft yarn comprises a multifilament configuration comprising a twisted yarn, a flat yarn, a textured yarn, or any combination thereof.

**Example 21:** The implantable prosthetic valve of any examples herein, particularly examples 14-19, wherein the plurality of courses are formed with a weft yarn, wherein the weft yarn is a monofilament yarn.

**Example 22:** The implantable prosthetic valve of any examples herein, particularly examples 14-19, wherein the plurality of courses are formed with a weft yarn, wherein the weft yarn comprises a composite construction in the form of a covered yarn.

**Example 23:** The implantable prosthetic valve of any examples herein, particularly examples 20-22, wherein the weft yarn is a combination of the twisted yarn or the flat yarn with the textured yarn.

**Example 24:** The implantable prosthetic valve of any examples herein, particularly examples 21-23, wherein the twisted yarn and/or flat yarn have a size of about 10 denier to about 40 denier, and wherein the textured yarn has a size of about 20 denier to about 160 denier.

**Example 25:** The implantable prosthetic valve of any examples herein, particularly example 23 or 24, wherein the weft yarn has a filament count from about 10 to about 200.

**Example 26:** The implantable prosthetic valve of any examples herein, particularly examples 20-25, wherein the textured yarn is configured to fill gaps in the mesh layer.

**Example 27:** The implantable prosthetic valve of any examples herein, particularly examples 17-26, wherein the warp yarn comprises a polyester, co-polyester, polyamide, polyolefin, polyaryletherketones, aromatic polymers, polyurethane, or any combination thereof.

Example 28: The implantable prosthetic valve of any examples herein, particularly examples 17-27, wherein the warp yarn comprises polyethylene terephthalate (PET), polytetrafluoroethylene (PTFE), Nylon, UHMWPE, PEEK, Liquid Crystalline Polymer, thermoplastic polyurethane (TPU), or a combination thereof.

Example 29: The implantable prosthetic valve of any examples herein, particularly examples 17-28, wherein the warp yarn comprises a polyester, co-polyester, polyamide, polyolefin, polyaryletherketones, aromatic polymers, polyurethane, or any combination thereof.

Example **30:** The implantable prosthetic valve of any examples herein, particularly examples 20-29, wherein the weft yarn comprises polyethylene terephthalate (PET), polytetrafluoroethylene (PTFE), Nylon, UHMWPE, PEEK, Liquid Crystalline Polymer, thermoplastic polyurethane (TPU), or a combination thereof.

Example **31:** The implantable prosthetic valve of any examples herein, particularly examples 8-28, wherein the mesh layer comprises a knit or woven fabric.

**Example 32:** The implantable prosthetic valve of any examples herein, particularly example 31, wherein the knit fabric is crochet knit and/or warp-knit fabric.

**Example 33:** The implantable prosthetic valve of any examples herein, particularly examples 8-32, wherein the pile yarns are arranged to form a looped pile.

**Example 34:** The implantable prosthetic valve of any examples herein, particularly examples 8-33, wherein the pile yarns are cut to form a cut pile.

**Example 35:** The implantable prosthetic valve of any examples herein, particularly examples 8-34, wherein a height of the pile yarns is substantially the same along the length and/or width of the outer sealing member.

**Example 36:** The implantable prosthetic valve of any examples herein, particularly examples 8-31, wherein a height of the pile yarns varies along the width of the outer sealing member.

**Example 37:** The implantable prosthetic valve of any examples herein, particularly examples 8-36, wherein the pile yarn comprises a flat or textured yarn.

**Example 38:** The implantable prosthetic valve of any examples herein, particularly examples 8-37, wherein the pile yarn has a size from about 20 denier to about 80 denier.

**Example 39:** The implantable prosthetic valve of any examples herein, particularly examples 8-38, wherein the pile yarn has a filament count from about 10 to about 100.

**Example 40:** The implantable prosthetic valve of any examples herein, particularly examples 8-39, wherein the pile yarn comprises a polyester, co-polyester, polyamide, polyolefin, polyaryletherketones, aromatic polymers, polyurethane, or any combination thereof.

**Example 41:** The implantable prosthetic valve of any examples herein, particularly examples 38-40, wherein the pile yarn comprises polyethylene terephthalate (PET), polytetrafluoroethylene (PTFE), Nylon, UHMWPE, PEEK, Liquid Crystalline Polymer, thermoplastic polyurethane (TPU), or a combination thereof.

**Example 42:** The implantable prosthetic valve of any examples herein, particularly examples 8-41, wherein the mesh layer comprises a woven fabric layer, and the pile layer comprises a separate pile layer that is stitched to the woven fabric layer.

**Example 43:** The implantable prosthetic valve of any examples herein, particularly examples 8-42, wherein the outer sealing member has an uncompressed thickness of about 0.6 mm to about 2.5 mm.

**Example 44:** The implantable prosthetic valve of any examples herein, particularly examples 8-43, wherein the outer sealing member has a compressed thickness of about 0.4 mm to about 1 mm, wherein the outer sealing member is compressed at 23.4+/-0.7 kPa.

**Example 45:** The implantable prosthetic valve of any examples herein, particularly examples 20-44, wherein the distal and the proximal end of the outer sealing member comprises at least two threads of the weft yarn.

**Example 46:** The implantable prosthetic valve of any examples herein, particularly examples 8-45, wherein the distal end and/or the outer end of the outer sealing member are laser cut.

**Example 47:** The implantable prosthetic valve of any examples herein, particularly examples 1-46, further comprising an inner skirt mounted on the inner surface of the annular frame, the inner skirt having an inflow end portion that is secured to the proximal end of the outer sealing member.

**Example 48:** The implantable prosthetic valve of any examples herein, particularly example 47, wherein the inflow end portion of the inner skirt is wrapped around the inflow end of the frame and overlaps the proximal end portion of the outer sealing member on the outside of the frame.

**Example 49:** The implantable prosthetic valve of any examples herein, particularly examples 1-48, wherein the implantable prosthetic valve is a heart valve.

**Example 50:** A method of forming an implantable prosthetic valve comprising: a) providing an annular frame having an inner surface and an outer surface wherein the frame has an inflow end and an outflow end; wherein the annular frame is compressible and expandable between a radially compressed configuration and a radially expanded configuration; b) circumferentially mounting an outer sealing member having a proximal end and a distal end around a first portion of the outer surface of the annular frame, wherein the first portion of the outer surface has a proximal end, and a distal end, wherein the proximal end of the first portion is at the inflow end of the annular frame, and wherein the first portion has a first length extending between the proximal end and the distal end in the radially compressed configuration of the annular frame and a second length extending between the proximal end and the distal end in the radially expanded configuration of the annular frame, wherein the outer sealing member has a length substantially identical to the first length of the first portion; and wherein the outer sealing member is configured to outwardly bulge when the annular frame is in the radially expanded configuration, thereby forming a seal against surrounding tissue when the prosthetic valve is implanted.

**Example 51:** The method of any examples herein, particularly example 50, wherein prior to the mounting, the annular frame is radially expanded to the expanded configuration.

**Example 52:** The method of any examples herein, particularly example 50 or 51, wherein the outer sealing member is substantially straightened along the first portion of the outer surface of the annular frame when the annular frame is in the radially compressed configuration.

**Example 53:** The method of any examples herein, particularly examples 50-52, wherein the outer surface of the annular frame has a second portion that is free of the outer sealing member and wherein the second portion extends between the outflow end of the annular frame and the distal end of the first portion.

**Example 54:** The method of any examples herein, particularly examples 50-53, wherein the proximal end of the outer sealing member is coupled to the proximal end of the first portion, and the distal end of the outer sealing member is coupled to the distal end of the first portion.

**Example 55:** The method of any examples herein, particularly example 54, wherein the outer sealing member is coupled to the proximal end and the distal end of the first portion by a fastener.

**Example 56:** The method of any examples herein, particularly example 55, wherein the fastener comprises sutures, pins, rivets, ultrasonic welding, laser welding, adhesive bonding, or any combination thereof.

**Example 57:** The method of any examples herein, particularly examples 50-56, wherein the outer sealing member is knitted or woven.

**Example 58:** The method of any examples herein, particularly example 57, wherein the outer sealing member is knitted, it is crochet knitted or warp-knitted.

**Example 59:** The method of any examples herein, particularly example 55 or 56, wherein the outer sealing member is knitted to the desired width.

**Example 60:** The method of any examples herein, particularly example 59, wherein the desired width is substantially similar to a circumference of the annular frame in the expanded configuration.

**Example 61:** The method of any examples herein, particularly examples 57-59, wherein the outer sealing member is laser cut at the proximal end and/or distal end.

**Example 62:** The method of any examples herein, particularly examples 50-61, wherein the outer sealing member comprises a mesh layer and a pile layer, wherein the pile layer comprises a plurality of pile yarns extending outwardly from at least a portion of an outer surface of the mesh layer, and wherein an inner surface of the mesh layer is substantially free of the pile yarns and wherein at least a portion of the inner surface of the mesh layer is in substantial contact with at least a portion of the outer surface of the annular frame.

**Example 63:** The method of any examples herein, particularly example 62, wherein the mesh layer has a first height extending axially along the frame, and the pile layer comprises a second height extending axially along the frame, wherein the first height is greater than the second height.

**Example 64:** The method of any examples herein, particularly example 62 or 63, wherein a portion of the outer surface of the mesh layer at the proximal end of the outer sealing member is substantially free of the pile yarn.

**Example 65:** The method of any examples herein, particularly examples 62-64, wherein a portion of the outer surface of the mesh layer at the distal end of the outer sealing member is substantially free of the pile yarn.

**Example 66:** The method of any examples herein, particularly examples 64-665, wherein the portion of the outer surface of the mesh layer at the proximal end of the outer sealing member that is substantially free of the pile yarn is sutured to the proximal end of the first portion of the annular frame.

**Example 67:** The method of any examples herein, particularly examples 65-66, wherein the portion of the outer surface of the mesh layer at the distal end of the outer sealing member that is substantially free of the pile yarn is sutured to the distal end of the first portion of the annular frame.

**Example 68:** The method of any examples herein, particularly examples 62-67, wherein the mesh layer comprises a plurality of warp and weft yarns and comprises a plurality of wales extending along axially across the length of the outer sealing layer and a plurality of courses extending circumferentially along the width of the outer sealing layer.

**Example 69:** The method of any examples herein, particularly example 68 wherein the mesh layer has a wales density from about 10 to about 50 wales per inch.

**Example 70:** The method of any examples herein, particularly example 68 or 69, wherein the mesh layer has a courses density from about 25 to about 85 courses per inch.

**Example 71:** The method of any examples herein, particularly examples 68-70, wherein the plurality of wales comprises a warp yarn, wherein the warp yarn is fully-drawn, or spin-drawn or low or not twisted.

**Example 72:** The method of any examples herein, particularly example 71, wherein the warp yarn has a size from about 10 deniers to about 40 deniers and a filament count from about 6 to about 56.

**Example 73:** The method of any examples herein, particularly example 71 or 72, wherein the warp yarn has a tenacity from about 30 to about 400 cN/tex.

**Example 74:** The method of any examples herein, particularly examples 68-73, wherein the plurality of courses are formed with a weft yarn, wherein the weft yarn comprises a multifilament configuration comprising a twisted yarn, a flat yarn, a textured yarn, or any combination thereof.

**Example 75:** The method of any examples herein, particularly examples 68-74, wherein the plurality of courses are formed with a weft yarn, wherein the weft yarn is a monofilament yarn.

**Example 76:** The method of any examples herein, particularly examples 68-73, wherein the plurality of courses are formed with a weft yarn, wherein the weft yarn comprises a composite construction in the form of a covered yarn.

**Example 77:** The method of any examples herein, particularly examples 74-76, wherein the weft yarn is a combination of the twisted yarn or the flat yarn with the textured yarn.

**Example 78:** The method of any examples herein, particularly examples 75-77, wherein the twisted yarn and/or flat yarn have a size of about 10 deniers to about 40 deniers, and wherein the textured yarn has a size of about 20 deniers to about 160 deniers.

**Example 79:** The method of any examples herein, particularly example 77 or 78, wherein the weft yarn has a filament count from about 10 to about 200.

**Example 80:** The method of any examples herein, particularly examples 74-79, wherein the textured yarn is configured to fill gaps in the mesh layer.

**Example 81:** The method of any examples herein, particularly examples 71-80, wherein the warp yarn comprises a polyester, co-polyester, polyamide, polyolefin, polyaryletherketones, aromatic polymers, polyurethane, or any combination thereof.

**Example 82:** The method of any examples herein, particularly examples 71-80, wherein the warp yarn comprises polyethylene terephthalate (PET), polytetrafluoroethylene (PTFE), Nylon, UHMWPE, PEEK, Liquid Crystalline Polymer, thermoplastic polyurethane (TPU), or a combination thereof.

**Example 83:** The method of any examples herein, particularly examples 74-82, wherein the weft yarn comprises **a** polyester, co-polyester, polyamide, polyolefin, polyaryletherketones, aromatic polymers, polyurethane, or any combination thereof.

**Example 84:** The method of any examples herein, particularly examples 74-83, wherein the weft yarn comprises polyethylene terephthalate (PET), polytetrafluoroethylene (PTFE), Nylon, UHMWPE, PEEK, Liquid Crystalline Polymer, thermoplastic polyurethane (TPU) or a combination thereof.

**Example 85:** The method of any examples herein, particularly examples 62-84, wherein the pile yarns are arranged to form a looped pile.

**Example 86:** The method of any examples herein, particularly examples 62-85, wherein the pile yarns are cut to form a cut pile.

**Example 87: The** method of any examples herein, particularly examples 62Fehler! Verweisquelle **konnte nicht gefunden werden.-86,** wherein a height of the pile yarns is substantially the same along the length and/or width of the outer sealing member.

**Example 88:** The method of any examples herein, particularly examples 62-87, wherein a height of the pile yarns varies along the width of the outer sealing member.

**Example 89:** The method of any examples herein, particularly examples 62-88, wherein the pile yarn comprises flat or textured yarn.

**Example 90:** The method of any examples herein, particularly examples 62-89, wherein the pile yarn has a size from about 20 deniers to about 80 deniers.

**Example 91:** The method of any examples herein, particularly examples 62-90, wherein the pile yarn has a filament count from about 10 to about 100.

**Example 92:** The method of any examples herein, particularly examples 62-91, wherein the pile yarn comprises a polyester, co-polyester, polyamide, polyolefin, polyaryletherketones, aromatic polymers, polyurethane, or any combination thereof.

**Example 93:** The method of any examples herein, particularly examples 62-92, wherein the pile yarn comprises polyethylene terephthalate (PET), polytetrafluoroethylene (PTFE), Nylon, UHMWPE, PEEK, Liquid Crystalline Polymer, thermoplastic polyurethane (TPU) or a combination thereof.

**Example 94:** The method of any examples herein, particularly examples 62-93, wherein the mesh layer comprises a woven fabric layer, and the pile layer comprises a separate pile layer that is stitched to the woven fabric layer.

**Example 95:** The method of any examples herein, particularly examples 62-94, wherein the outer sealing member has an uncompressed thickness of about 0.6 mm to about 2.5 mm.

**Example 96:** The method of any examples herein, particularly examples 62-95, wherein the outer sealing member has a compressed thickness of about 0.4 mm to about 1 mm, wherein the outer sealing member is compressed at 23.4+/-0.7 kPa.

**Example 97:** The method of any examples herein, particularly examples 62-96, wherein the distal and the proximal end of the outer sealing member comprises at least two threads of the weft yarn.

**Example 98:** The method of any examples herein, particularly examples 62-97, wherein the distal end and/or the outer end of the outer sealing member are laser cut.

**Example 99:** The method of any examples herein, particularly examples 50-98, further comprising an inner skirt mounted on the inner surface of the annular frame, the inner skirt having an inflow end portion that is secured to the proximal end of the outer sealing member.

**Example 100:** The method of any examples herein, particularly example 99, wherein the inflow end portion of the inner skirt is wrapped around the inflow end of the frame and overlaps the proximal end portion of the outer sealing member on the outside of the frame.

**Example 101:** The method of any examples herein, particularly examples 50-100, wherein the implantable prosthetic valve is a heart valve.

In the following the further embodiments are disclosed:
1. An implantable prosthetic valve comprising:
   a) an annular frame having an inner surface and an outer surface, an inflow end, and an outflow end;
      wherein the annular frame is compressible and expandable between a radially compressed configuration and a radially expanded configuration; and
   b) an outer sealing member having a proximal end and a distal end and is mounted circumferentially around a first portion of the outer surface of the annular frame, wherein the first portion of the outer surface has a proximal end and a distal end, wherein the proximal end of the first portion is at the inflow end of the annular frame, and wherein the first portion has a first length extending between the proximal end and the distal end in the radially compressed configuration of the annular frame and a second length extending between the proximal end and the distal end in the radially expanded configuration of the annular frame,

   wherein the outer sealing member has a length substantially identical to the first length of the first portion; and
   wherein the outer sealing member is configured to outwardly bulge when the annular frame is in the radially expanded configuration, thereby forming a seal against surrounding tissue when the prosthetic valve is implanted.
2. The implantable prosthetic valve of embodiment 1, wherein the outer surface of the annular frame has a second portion that is free of the outer sealing member and wherein the second portion extends between the outflow end of the annular frame and the distal end of the first portion.
3. The implantable prosthetic valve of any one of embodiments 1-2, wherein the outer sealing member comprises a mesh layer and a pile layer, wherein the pile layer comprises a plurality of pile yarns extending outwardly from at least a portion of an outer surface of the mesh layer, wherein an inner surface of the mesh layer is substantially free of the pile yarns and wherein at least a portion of the inner surface of the mesh layer is in substantial contact with at least a portion of the outer surface of the annular frame.
4. The implantable prosthetic valve of embodiment 3, wherein the mesh layer has a first height extending axially along the frame, and the pile layer has a second height extending axially along the frame, wherein the first height is greater than the second height.
5. The implantable prosthetic valve of any one of embodiments 3-4, wherein the mesh layer comprises a plurality of warp and weft yarns and comprises a plurality of wales extending axially across the length of the outer sealing layer and a plurality of courses extending circumferentially along a width of the outer sealing layer, wherein the width of the outer layer is substantially identical to a circumference of the annular frame in the expanded configuration.
6. The implantable prosthetic valve of embodiment 5, wherein the mesh layer has a wales density from about 10 to about 50 wales per inch and/or a course density from about 25 to about 85 courses per inch.
7. The implantable prosthetic valve of any one of embodiments 5-6, wherein the plurality of wales comprises a warp yarn, wherein the warp yarn is fully-drawn, or spin-drawn or low or not twisted.
8. The implantable prosthetic valve of embodiment 7, wherein the warp yarn has a size from about 10 denier to about 40 denier and a filament count from about 6 to about 56 and/or wherein the warp yarn has a tenacity from about 30 to about 400 cN/tex.
9. The implantable prosthetic valve of any one of embodiments 5-8, wherein the plurality of courses are formed with a weft yarn, wherein the weft yarn comprises a multifilament configuration comprising a twisted yarn, a flat yarn, a textured yarn, or any combination thereof.
10. The implantable prosthetic valve of any one of embodiments 5-8, wherein the plurality of courses are formed with a weft yarn, wherein the weft yarn is a monofilament yarn.
11. The implantable prosthetic valve of any one of embodiments 5-8, wherein the plurality of courses are formed with a weft yarn, wherein the weft yarn comprises a composite construction in the form of a covered yarn.
12. The implantable prosthetic valve of any one of embodiments 9-11, wherein the weft yarn is a combination of the twisted yarn or the flat yarn with the textured yarn.
13. The implantable prosthetic valve of any one of embodiments 10-12, wherein the twisted yarn and/or flat yarn have a size of about 10 denier to about 40 denier, and wherein the textured yarn has a size of about 20 denier to about 160 denier.
14. The implantable prosthetic valve of any one of embodiments 9-13, wherein the textured yarn is configured to fill gaps in the mesh layer.
15. The implantable prosthetic valve of any one of embodiments 7-14, wherein the warp yarn comprises a polyester, co-polyester, polyamide, polyolefin, polyaryletherketones, aromatic polymers, polyurethane, or any combination thereof.
16. The implantable prosthetic valve of any one of embodiments 7-15, wherein the weft yarn comprises a polyester, co-polyester, polyamide, polyolefin, polyaryletherketones, aromatic polymers, polyurethane, or any combination thereof.
17. The implantable prosthetic valve of any one of embodiments 3-16, wherein the mesh layer comprises a knit or woven fabric.
18. The implantable prosthetic valve of embodiment 17, wherein the knit fabric is crochet knit and/or warp-knit fabric.
19. The implantable prosthetic valve of any one of embodiments 3-18, wherein the pile yarns are arranged to form a looped pile or wherein the pile yarns are cut to form a cut pile.
20. The implantable prosthetic valve of any one of embodiments 3-19, wherein the pile yarn comprises a flat or textured yarn.

## Claims

1. A method of forming an implantable prosthetic valve (10) comprising:
a) providing an annular frame (12) having an inner surface and an outer surface; wherein the annular frame (12) has an inflow end (15) and an outflow end (19); wherein the annular frame (12) is compressible and expandable between a radially compressed configuration and a radially expanded configuration;
b) circumferentially mounting an outer sealing member (18) having a proximal end (1802) and a distal end (1804) around a first portion (1806) of the outer surface of the annular frame (12), wherein the first portion (1806) of the outer surface has a proximal end (1806a), and a distal end (1806b), wherein the proximal end (1806a) of the first portion is at the inflow end (15) of the annular frame (12), and wherein the first portion (1806) has a first length extending between the proximal end (1806a) and the distal (1806b) end in the radially compressed configuration of the annular frame (12) and a second length extending between the proximal end (1806a) and the distal end (1806b) in the radially expanded configuration of the annular frame (12), wherein the outer sealing member (18) comprises a mesh layer (170) and a pile layer (172), wherein the pile layer (172) comprises a plurality of pile yarns (174) extending outwardly from at least a portion of an outer surface of the mesh layer (170), and wherein an inner surface of the mesh layer (170) is substantially free of the pile yarns (174), and wherein at least a portion of the inner surface of the mesh layer i(170) s in substantial contact with at least a portion of the outer surface of the annular frame (12); wherein the outer sealing member (18) has a length substantially identical to the first length of the first portion (1806); and
wherein the outer sealing member (18) is configured to outwardly bulge when the annular frame (12) is in the radially expanded configuration.

2. The method according to claims 1, wherein the outer surface of the annular frame (12) has a second portion (1820) that is free of the outer sealing member (18) and wherein the second portion (1820) extends between the outflow end (19) of the annular frame (12) and the distal end (1806b) of the first portion (1806).

3. The method according to any one of claims 1 or 2, wherein the mesh layer (170) has a first height extending axially along the annular frame (12), and the pile layer (172) comprises a second height extending axially along the annular frame (12), wherein the first height is greater than the second height.

4. The method according to any one of claims 1 to 3, wherein the mesh layer (170) comprises a plurality of warp and weft yarns and comprises a plurality of wales (1702) extending along axially across the length of the outer sealing layer (18) and a plurality of courses extending circumferentially along the width of the outer sealing layer (18).

5. The method according to claim 4, wherein the mesh layer (170) has a wales density from about 10 to about 50 wales per inch.

6. The method according to claims 4 or 5, wherein the plurality of wales (1702) comprises a warp yarn, wherein the warp yarn is fully-drawn, or spin-drawn or low or not twisted.

7. The method according to claim 6, wherein the warp yarn has a size from about 10 deniers to about 40 deniers and a filament count from about 6 to about 56 and/or wherein the warp yarn has a tenacity from about 30 to about 400 cN/tex.

8. The method according to any one of claims 4 to 7, wherein the plurality of courses are formed with a weft yarn, wherein the weft yarn comprises a multifilament configuration comprising a twisted yarn, a flat yarn (640), a textured yarn (660), or any combination thereof.

9. The method according to any one of claims 4 to 7, wherein the plurality of courses are formed with a weft yarn, wherein the weft yarn is a monofilament yarn; or wherein the plurality of courses are formed with a weft yarn, wherein the weft yarn comprises a composite construction in the form of a covered yarn.

10. The method according to any one of claims 8 or 9, wherein the weft yarn is a combination of the twisted yarn or the flat yarn (640) with the textured yarn (660).

11. The method of any of claims 9 or 10, wherein the twisted yarn and/or flat yarn (640) have a size of about 10 deniers to about 40 deniers, and wherein the textured yarn (660) has a size of about 20 deniers to about 160 deniers.

12. The method of any of claims 8 to 10, wherein the textured yarn (660) is configured to fill gaps in the mesh layer (170).

13. The method according to any one of claims 7 to 12, wherein the warp yarn comprises a polyester, co-polyester, polyamide, polyolefin, polyaryletherketones, aromatic polymers, polyurethane, or any combination thereof; and/or wherein the weft yarn comprises a polyester, co-polyester, polyamide, polyolefin, polyaryletherketones, aromatic polymers, polyurethane, or any combination thereof.

14. The method according to any one of the preceding claims, wherein the mesh layer (170) comprises a woven fabric layer, and the pile layer (172) comprises a separate pile layer (172) that is stitched to the woven fabric layer; and/or wherein the outer sealing member (18) is knitted, it is crochet knitted or warp-knitted.

15. The method according to any one of the preceding claims, wherein the pile yarns (174) are arranged to form a looped pile (176) or wherein the pile yarns (174) are cut to form a cut pile; and/or wherein the pile yarn (174) comprises flat (640) or textured yarn (660).
